# EUROPEAN PATENT APPLICATION

(11) **EP 4 817 606 A1**
(43) Date of publication of application: **07.10.2026**
(21) Application number: 25167343.0
(22) Date of filing: 31.03.2025
(51) Int. Cl.: A61K 31/192, A61K 31/196, A61K 31/4164, A61K 31/4192, A61K 31/426, A61K 31/505, A61P 1/16, A61P 3/10, A61P 9/10, A61P 35/00

(54) **HNF4 MODULATORS AND THEIR USES IN THERAPY**

(71) Applicant: Johann-Wolfgang-Goethe-Universität Frankfurt am Main, 60323 Frankfurt am Main (DE); Ludwig-Maximilians-Universität München, 80539 München (DE)
(72) Inventor: MERK, Daniel, 80333 München (DE); SCHALLMAYER, Espen, 64291 Darmstadt (DE); SCHUBERT-ZSILAVECZ, Manfred, 61352 Bad Homburg (DE); MOROZOV, Vasily, 82131 Gauting (DE); MARSCHNER, Julian, 80809 München (DE)
(74) Representative: Patentanwälte Dr. Keller, Schwertfeger Partnerschaft mbB

(57) **Abstract**

The present invention relates to novel hepatocyte nuclear factor 4 (HNF4) modulators, in particular HNF4 agonists and HNF4 antagonists, and their use in the treatment or prevention of a disease that is associated with HNF4 malfunction or genetic mutations of the HNF4 gene such type 2 diabetes, maturity-onset diabetes of the young type 1 (MODY1), maturity-onset diabetes of the young type 3 (MODY3), neonatal hyperinsulinemic hypoglycemia, gastrointestinal disease, liver disease, pancreatic disease, gastrointestinal cancer, inflammatory bowel disease (IBD), atherosclerosis, metabolic dysfunction, metabolic syndrome, insulin resistance or lipid disorders. The invention also relates to pharmaceutical compositions comprising an HNF4 modulator of the invention, and in vitro methods of analyzing the effects of hepatocyte nuclear factor 4 (HNF4) activity in a biological system.

## Description

### Technical Field

The present invention relates to novel hepatocyte nuclear factor 4 (HNF4) modulators, in particular HNF4 agonists and HNF4 antagonists, and their use in the treatment or prevention of a disease that is associated with HNF4 malfunction or genetic mutations of the HNF4 gene such as type 2 diabetes, maturity-onset diabetes of the young type 1 (MODY1), maturity-onset diabetes of the young type 3 (MODY3), neonatal hyperinsulinemic hypoglycemia, gastrointestinal disease, liver disease, pancreatic disease, gastrointestinal cancer, inflammatory bowel disease (IBD), atherosclerosis, metabolic dysfunction, metabolic syndrome, insulin resistance or lipid disorders. The invention also relates to pharmaceutical compositions comprising an HNF4 modulator of the invention, and in vitro methods of analyzing the effects of HNF4 activity in a biological system.

### State of the Art

HNF4 is a ligand-activated transcription factor and an orphan member of the nuclear receptor family. It exists in two isoforms (HNF4α, HNF4y) which display conserved DNA and ligand binding domains (LBD) but vary in their N- and C-terminal regions and exhibit different expression patterns. The HNF4α isoform is expressed in liver, intestine, and pancreatic β-cells while the redundant paralog HNF4γ is exclusively found in the intestine. Both isoforms are obligate homodimers with constitutive transactivation activity. Furthermore, HNF4 controls hepatic transcription of genes involved in gluconeogenesis and lipid metabolism and acts as a master regulator of islet gene expression in the pancreas.

HNF4 binds fatty acids such as palmitic, myristic and linoleic acid as natural ligands with high affinity. However, the receptor is hardly explored from a medicinal chemistry perspective despite its therapeutic potential. Mutations of the HNF4α isoform cause the inheritable form of type 2 diabetes known as maturity-onset diabetes of the young 1 (MODY-1). Dysregulation of HNF4 is associated with gastrointestinal diseases, metabolic disorders, and gastrointestinal cancers. This critical role in metabolic homeostasis underscores remarkable therapeutic potential of HNF4 modulators in diseases associated with metabolic dysfunction. Experiments in mice suggested that modulation of the activity of HNF4α may render the capabilities of controlling lipid metabolism and glucose metabolism, and may be effectively used to develop an agent for preventing or improving hypertriglyceridemia, fatty liver and diabetes. Furthermore, several lines of evidence point to a role of HNF4 in other conditions and potential of HNF4 modulators in therapy:
The Japanese Patent Application No. 2008-133247 discloses nitrogenistein, which is an inhibitor of HNF4α activity for preventing or improving fatty liver or diabetes.

WO 2002/024227 A2 discloses that HNF4α protein is overexpressed in colorectal tissues and that inhibiting HNF4α protein can inhibit the proliferation of tumor cells.

US 2010/0286220 A1 discloses BIM5078 compound as a HNF4α antagonist and methods of use. HNF4α antagonists can bind HNF4α in the fatty acid binding pocket of HNF4α.

WO 2005/010202 A2 discloses HNF4α receptor modulator compounds and their use as pharmaceutical agents as well as methods of using these compounds for modulating HNF4α receptors. The compounds are based on phenyl acetic acid.

WO 2005/0009104 A2 also discloses HNF4α receptor modulator compounds and their uses as pharmaceutical agents as well as methods of using these compounds for modulating HNF4α receptors. The compounds are based on benzoic and phenyl acetic acid.

US 2017/0189371 A1 discloses HNF4α antagonists and their use thereof. The HNF4α antagonist can significantly reduce the expression of Wnt5a in a specific manner compared to that of conventional known HNF4α antagonists, and can also inhibit the growth of gastric cancer cells.

US 2024/0207204 A1 discloses compounds for the treatment of subjects having type 2 diabetes and/or chronic kidney disease using HNF4α antagonists. The HNF4α antagonists comprises alverine, N-trans caffeoyltyramine (NCT), or a small activating ribonucleic acid (saRNA).

Against this background, it is object of the present invention to provide alternative compounds that modulate HNF4 activity and that are therapeutically suitable for the use in the treatment or prevention of a disease that is associated with HNF4-malfunction or genetic mutations of the HNF4 gene.

This object is solved by a modulator of hepatocyte nuclear factor 4 (HNF4) described herein.

The present invention discloses novel compounds that function as HNF4 modulators. In a first aspect, the invention relates to HNF4 modulators which have the ability to modulate HNF4 function or activity. In some embodiments, the HNF4 modulators act as HNF4 agonists, while in other embodiments, the HNF4 modulators are HNF4 antagonists. The HNF4 modulators of the invention affect the induction or activity of HNF4 and its target genes. In preferred embodiments, the HNF4 modulator is an HNF4α agonist. In alternative embodiments, the HNF4 modulator is an HNF4γ agonist. In alternative embodiments, the HNF4 modulator is a HNF4α antagonist or a HNF4γ antagonist.

The compounds that were identified as suitable HNF4 modulators according to the present invention comprise one of the following generic chemical structures 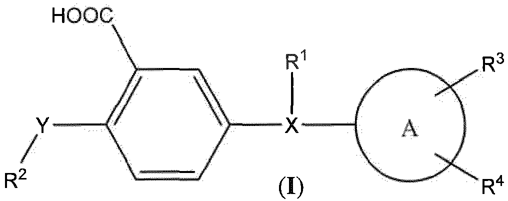 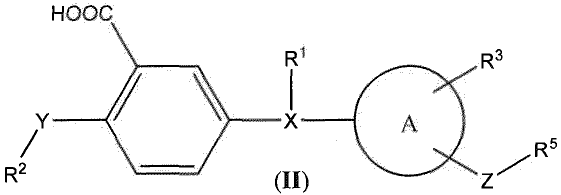 wherein
A is benzene or a heteroaromatic system,
X is C1-C5 alkyl, C3-C9 (bi)cycloalkyl, C2-C5 alkenyl, or C2-C5 alkynyl,
Y is -C(=O)-, -C(=O)NH-, -NHC(=O)-, -C-, -O-, -NH-, -N(alkyl)-, or -S-
Z is C1-C4 alkyl, -C(=O)-, -C(=O)NH-, -NHC(=O)-, -O-, -NH-, -N(alkyl)-, or -S-
R1 is -H, C1-C4 alkyl, or C1-C4 acyl;
R2 is -H, -OH, -NH(alkyl), C1-C4 alkyl, or C1-C4 acyl;
R3, R4, R5 is independently selected from -H, C1-C6 alkyl, C3-C6 cycloalkyl, C1-C6 haloalkyl, C1-C6 acyl, halo, phenyl optionally substituted, or heteroaryl optionally substituted;
R3 and R4 or R3 and R5 optionally form a cyclic system.

In preferred embodiments, the heteroaromatic system is selected from the group consisting of pyridine, pyrimidine, pyridazine, pyrazine, furan, thiophene, pyrrole, oxazole, isoxazole, imidazole, pyrazole, triazole, thiazole, isothiazole; X is C1-C5 alkyl, C3-C6 cycloalkyl, C4-C9 bicycloalkyl, C2-C5 alkenyl, C2-C5 alkynyl, each optionally containing -O-, -C(=O)-, -N(R1)-, or -C(=O)N(R1)-; R3 and R4 or R3 and R5 optionally form a cyclic system.

As used herein, the term "alkyl" denotes a hydrocarbon group or residue which is structurally similar to an alkane compound modified by the removal of one hydrogen from the non-cyclic alkane and the substitution therefore of a non-hydrogen moiety.

The term "cycloalkyl" denotes a hydrocarbon group or residue which is structurally similar to a cyclic alkane compound modified by the removal of one hydrogen from the cyclic alkane and substitution therefore of a non-hydrogen moiety. Cycloalkyls typically comprise a cyclic radical containing 3 to 8 ring carbons, such as cyclopropyl, cyclobutyl, cyclopentyl, cyclohexyl, cycloheptyl and the like. Cycloalkyl radicals can be multicyclic and can contain a total of 3 to 18 carbons, or preferably 4 to 12 carbons, or 5 to 8 carbons. Examples of multicyclic cycloalkyls include decahydronapthyl, adamantyl, and like radicals.

The term "haloalkyl" denotes an alkyl residue as defined above, substituted with one or more halogens, preferably fluorine, such as a trifluoromethyl, pentafluoroethyl and the like.

The term "alkenyl" denotes an alkyl residue as defined above that also comprises at least one carbon-carbon double bond in the backbone of the hydrocarbon chain. Examples include but are not limited to vinyl, allyl, 2-butenyl, 3-butenyl, 2-pentenyl, 3-pentenyl, 4-pentenyl, 2-hexenyl, 3-hexenyl, 4-hexenyl, 5-hexenyl, 2-heptenyl, 3-heptenyl, 4-heptenyl, 5-heptenyl, 6-heptenyl and the like. The term "alkenyl" includes dienes and trienes of straight and branch chains.

The term "acyl" denotes a R-C(O)- residue having an R group containing 1 to 8 carbons. Examples include but are not limited to formyl, acetyl, propionyl, butanoyl, isobutanoyl, pentanoyl, hexanoyl, heptanoyl, benzoyl and the like, and natural or un-natural amino acids.

The term "heteroaryl" denotes an aryl ring radical as defined above, wherein at least one of the ring carbons, or preferably 1, 2, or 3 carbons of the aryl aromatic ring has been replaced with a heteroatom, which include but are not limited to nitrogen, oxygen, and sulfur atoms. Examples of heteroaryl residues include pyridyl, bipyridyl, furanyl, and thiofuranyl residues. Substituted "heteroaryl" residues can have one or more organic or inorganic substituent groups, or preferably 1, 2, or 3 such groups, as referred to herein-above for aryl groups, bound to the carbon atoms of the heteroaromatic rings. The organic substituent groups can comprise from 1 to 12 carbon atoms, or from 1 to 6 carbon atoms, or from 1 to 4 carbon atoms.

The term "HNF4 agonists" denotes compounds that induce or activate HNF4 leading to the modulation of gene expression and subsequent physiological effects that are beneficial in the treatment of metabolic and inflammatory diseases. For example, when an HNF4α agonist binds to the HNF4α receptor, it induces a conformational change in the protein, enhancing its ability to interact with DNA response elements. This interaction promotes the transcription of target genes that are involved in various metabolic pathways.

The term "HNF4 antagonists" denotes compounds that competitively bind to HNF4 in the presence of natural or known HNF4 agonists.

"Optional" or "optionally" means that the subsequently described event or circumstance can or cannot occur, and that the description includes instances where the event or circumstance occurs and instances where it does not.

The terms "pharmaceutically acceptable" denote a material that is not biologically, clinically or otherwise undesirable, i.e., the material can be administered to an individual along with the relevant active compound without causing clinically unacceptable biological effects or interacting in a deleterious manner with any of the other components of the pharmaceutical composition in which it is contained.

The term "subject" denotes any animal, including mammals. Mammals include, but are not limited to farm animals (such as, for example, horses, cows, and pigs), companion animals (such as, for example, dogs and cats), laboratory animals (such as, for example, mice, rats, and rabbits), and non-human primates. In some embodiments, the subject is a human. In some embodiments, the human is a patient under the care of a physician.

The "effective amount" denotes a nontoxic but sufficient amount of the compound to provide the desired result. As will be pointed out below, the exact amount required will vary from subject to subject, depending on the species, age, and general condition of the subject, the severity of the disease that is being treated, the particular compound used, its mode of administration, and the like. Thus, it is not possible to specify an exact "effective amount." However, an appropriate effective amount can be determined by one of ordinary skill in the art using only routine experimentation.

Preferred HNF4-modulating compounds falling under the above generic formulas have one of the following structures:

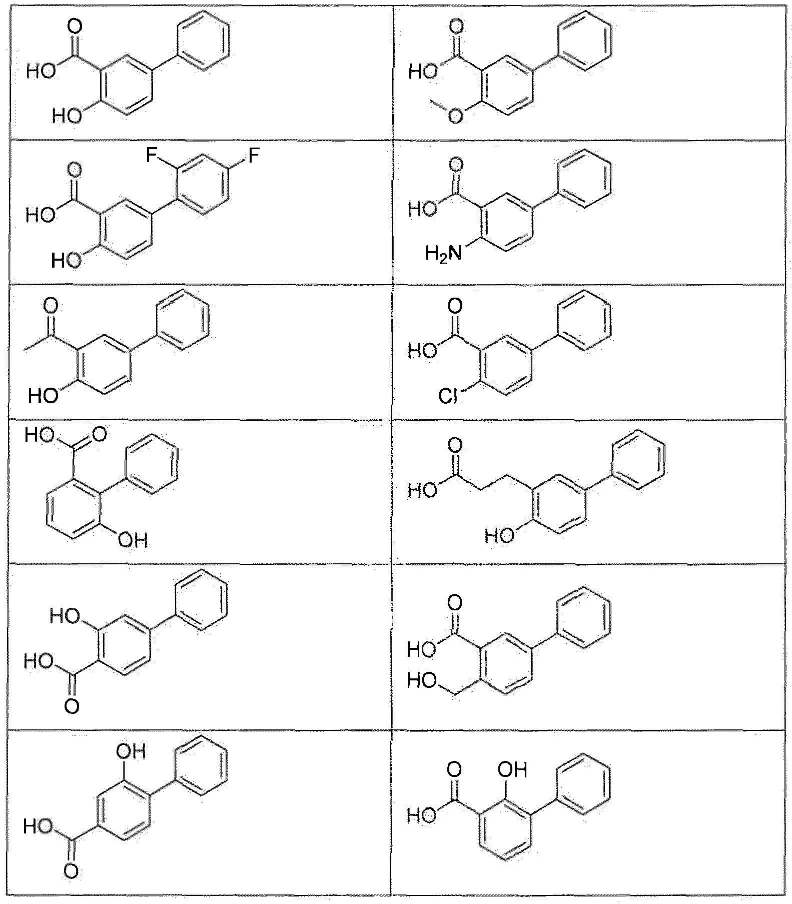

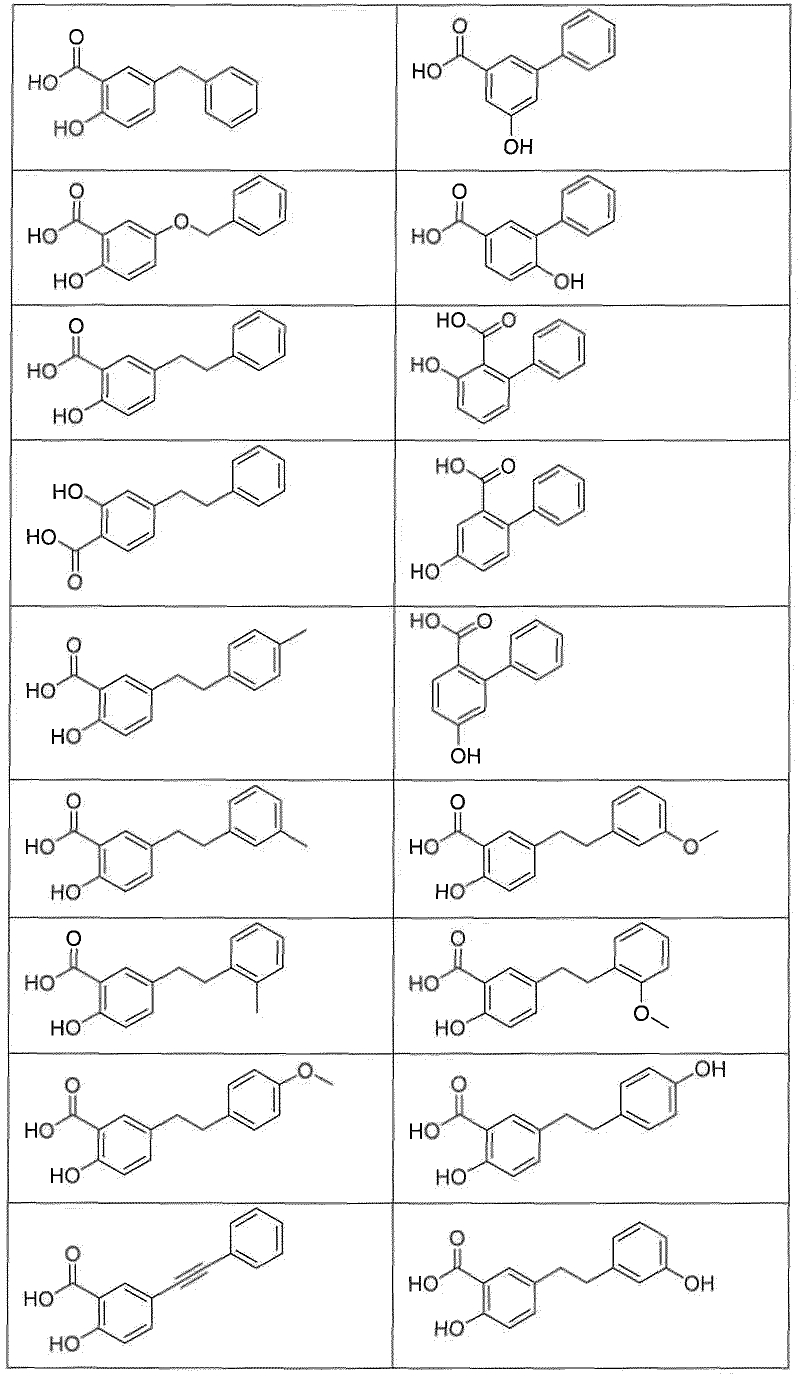

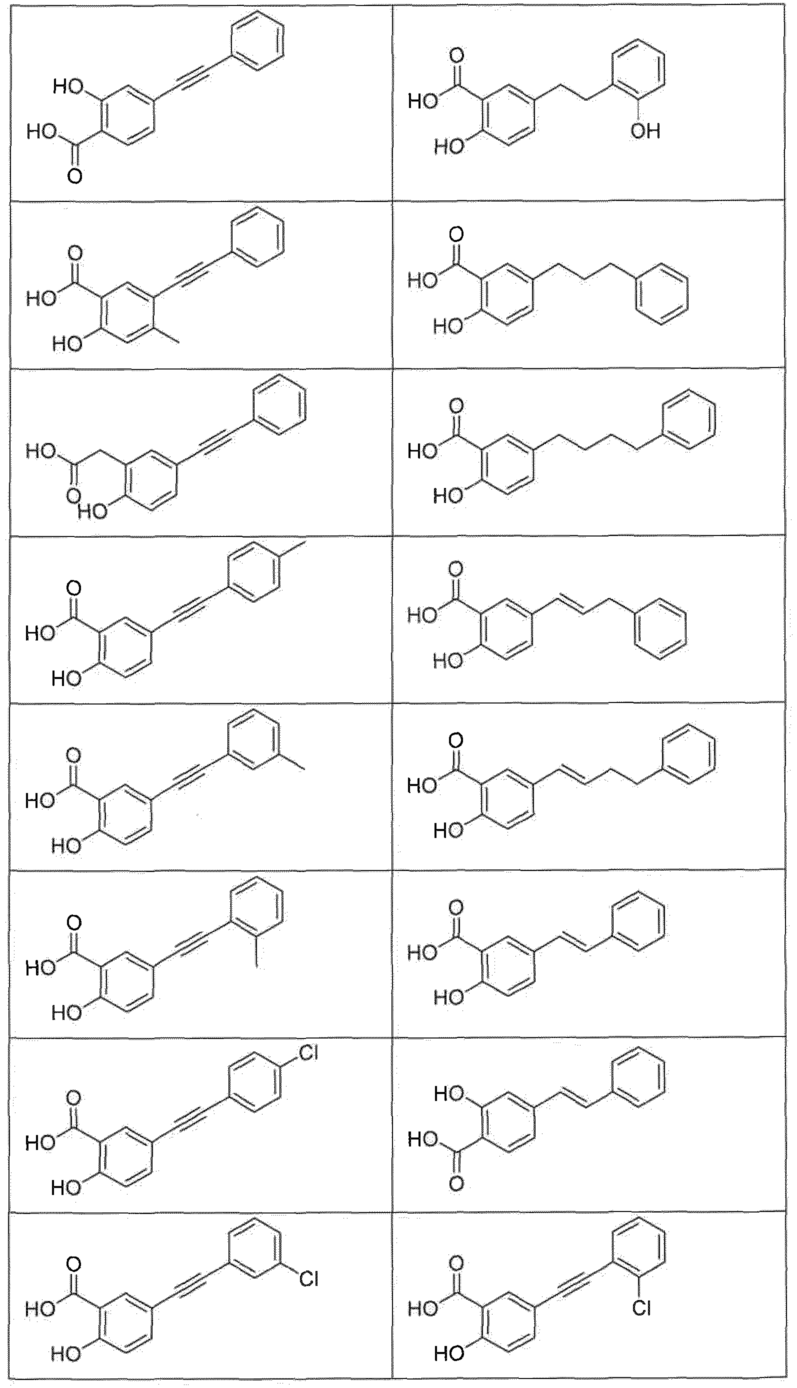

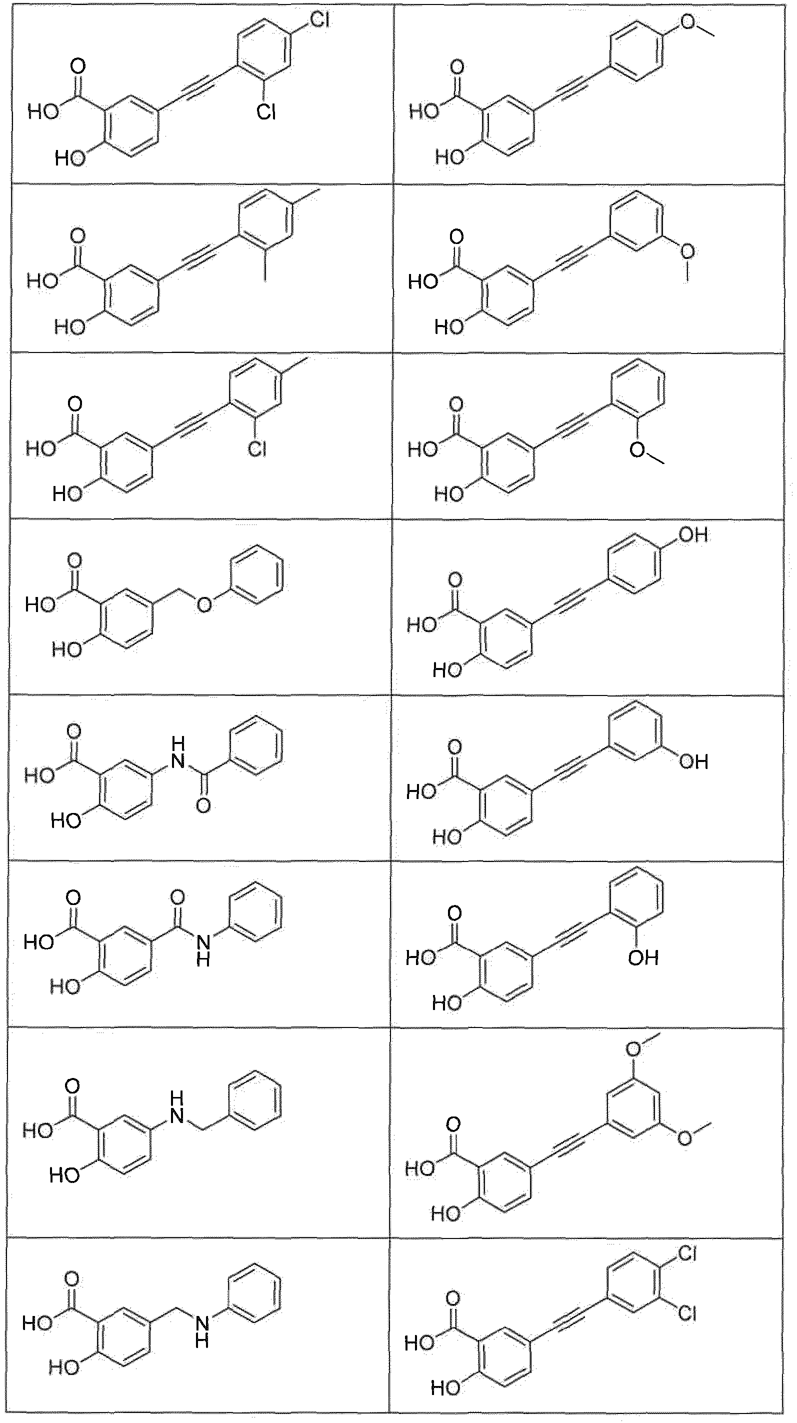

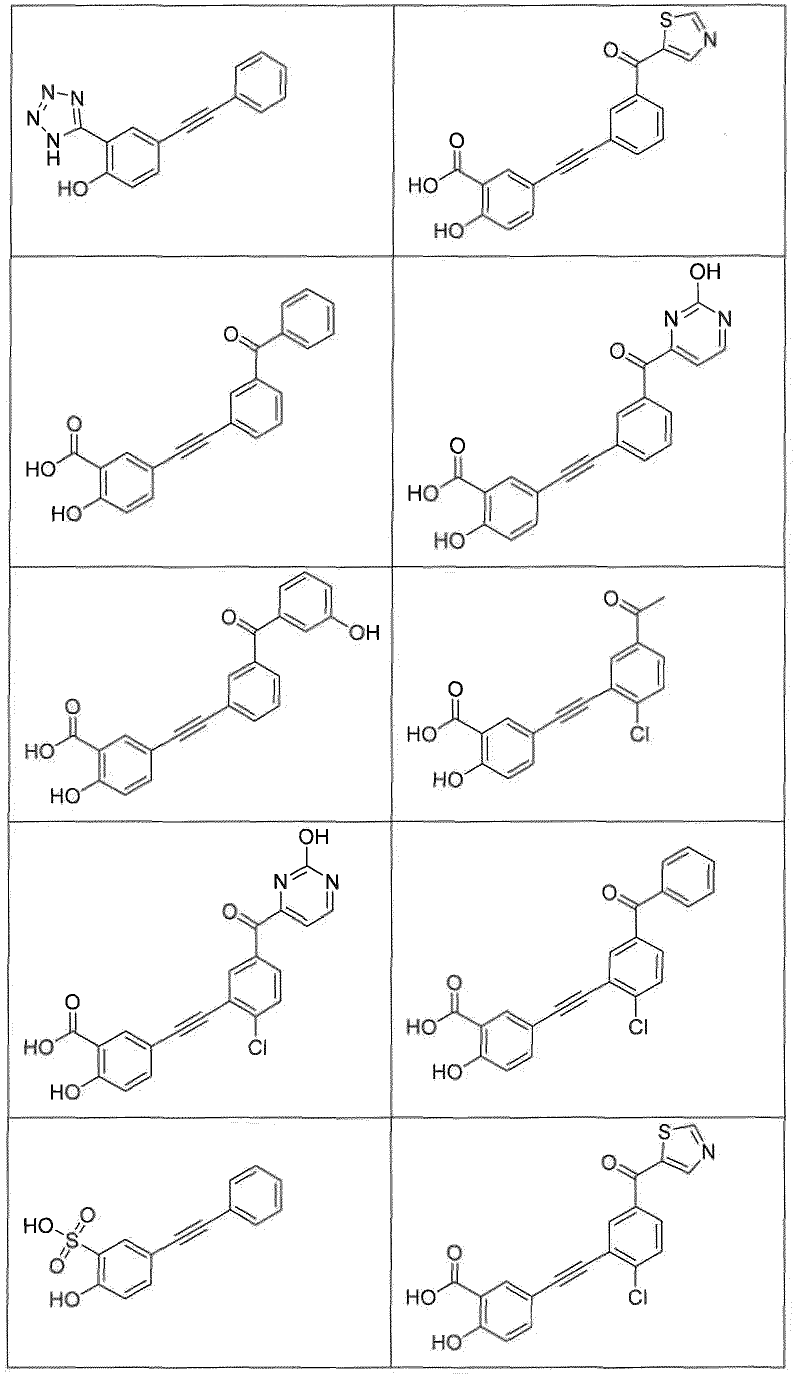

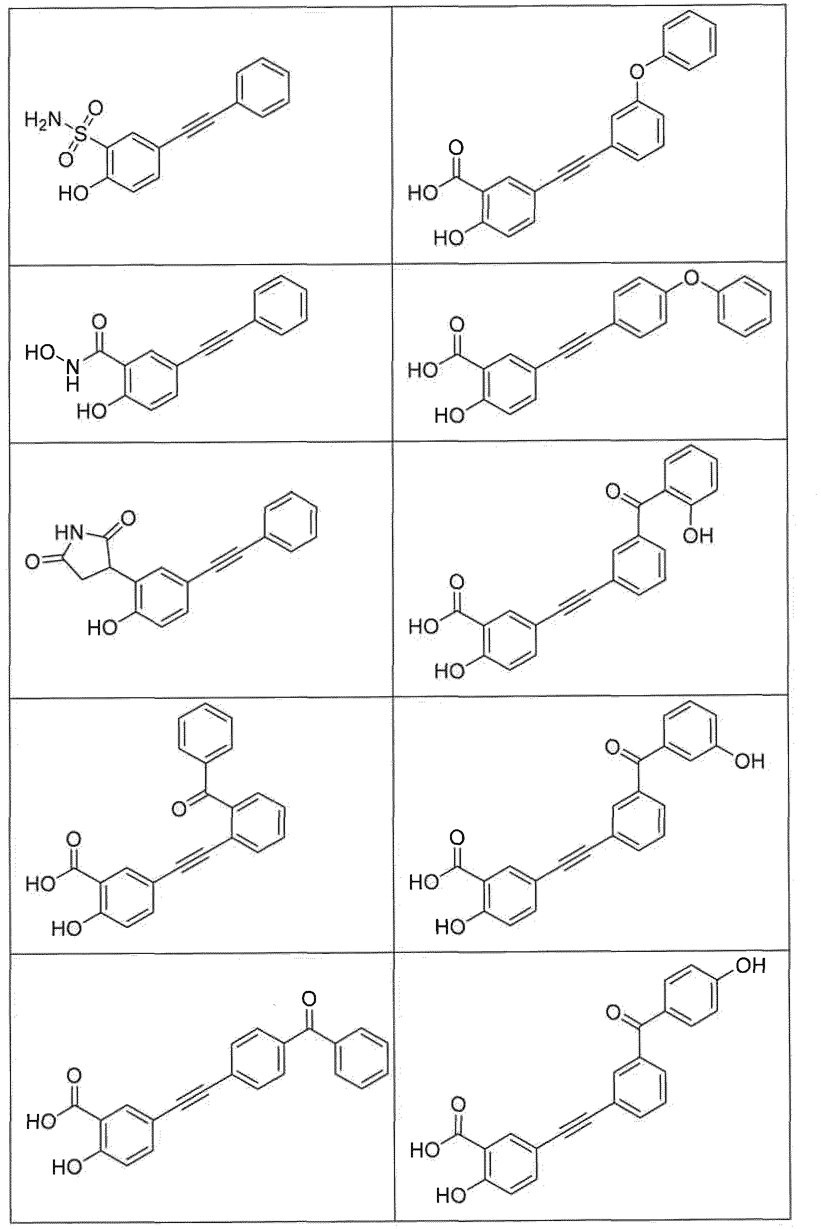

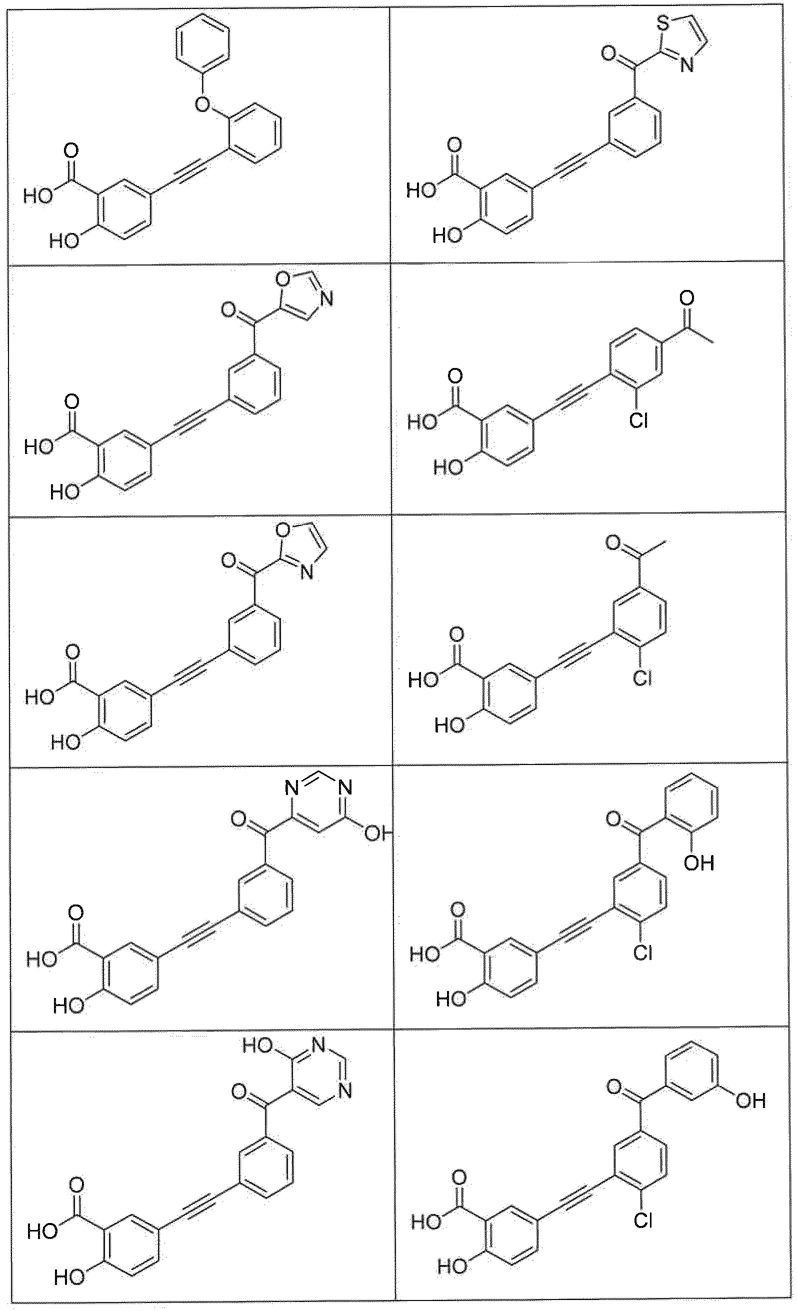

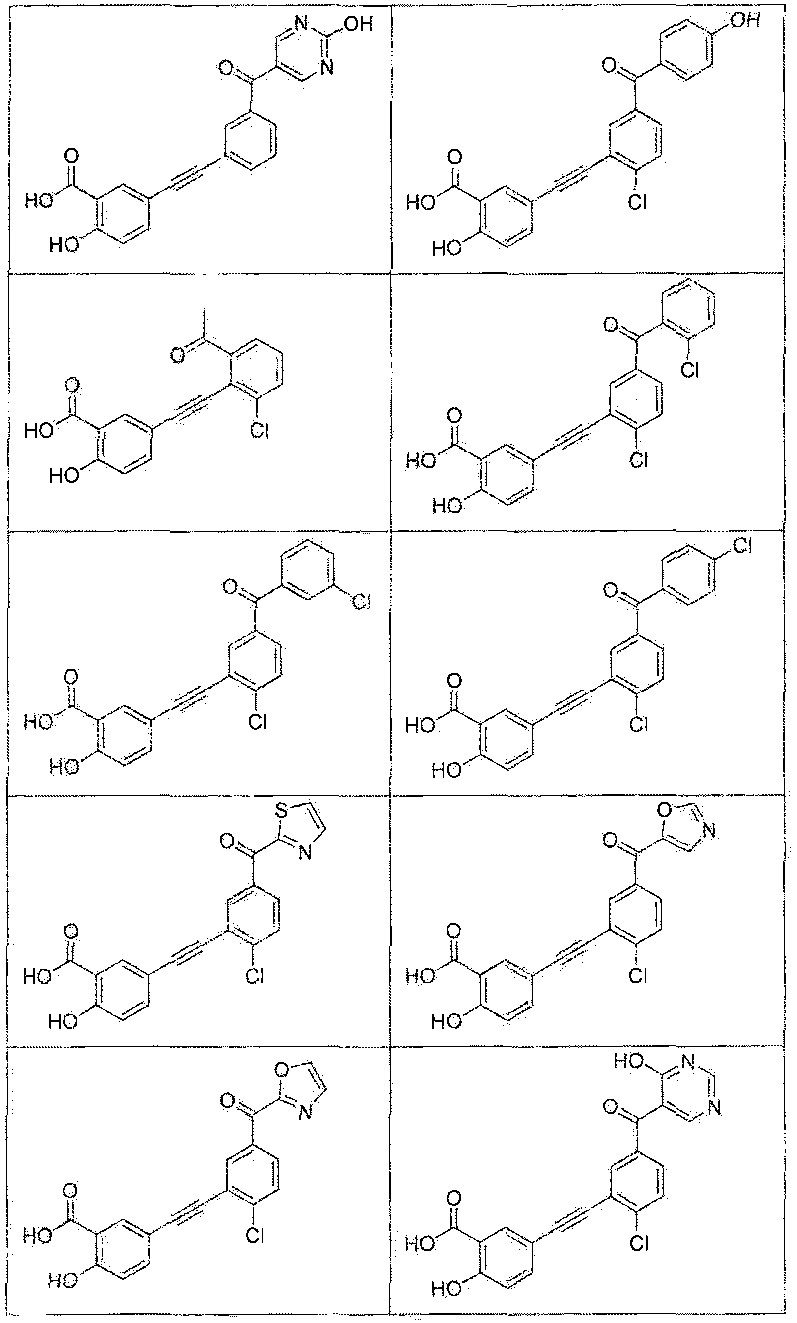

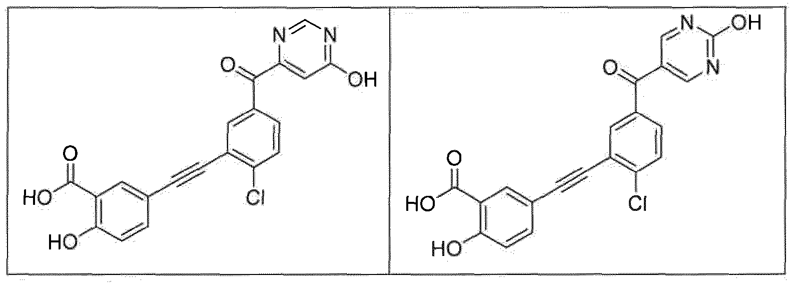

In some preferred embodiments, the HNF4 modulator is selected from the group consisting of
4-(Hydroxymethyl)[1,1'-biphenyl]-3-carboxylic acid (8)
6-Hydroxy[1,1'-biphenyl]-3-carboxylic acid (13)
3-Hydroxy[1,1'-biphenyl]-2-carboxylic acid (16)
2-Hydroxy-5-(3-phenylpropyl)benzoic acid (19)
2-Hydroxy-5-(4-phenylbutyl)benzoic acid (20)
(E)-2-Hydroxy-5-styrylbenzoic acid (22)
2-Hydroxy-4-phenethylbenzoic acid (24)
(E)-2-Hydroxy-4-styrylbenzoic acid (25)
2-Hydroxy-4-(phenylethynyl)benzoic acid (26)
2-Hydroxy-4-methyl-5-(phenylethynyl)benzoic ac-id (27)
2-Hydroxy-5-(4-methylphenylethynyl)benzoic acid (28)
5-[(4-Chlorophenyl)ethynyl]-2-hydroxybenzoic acid (29)
2-Hydroxy-5-[(4-methoxyphenyl)ethynyl]benzoic acid (30)
2-Hydroxy-5-[(4-hydroxyphenyl)ethynyl]benzoic acid (31)
2-Hydroxy-5-(3-methylphenylethynyl)benzoic acid (32)
5-[(3-Chlorophenyl)ethynyl]-2-hydroxybenzoic acid (33)
2-Hydroxy-5-[(3-methoxyphenyl)ethynyl}benzoic acid (34)
2-Hydroxy-5-[(3-hydroxyphenyl)ethynyl]benzoic acid (35)
2-Hydroxy-5-(2-methylphenylethynyl)benzoic acid (36)
5-[(2-Chlorophenyl)ethynyl]-2-hydroxybenzoic acid (37)
2-Hydroxy-5-[(2-methoxyphenyl)ethynyl]benzoic acid (38)
2-Hydroxy-5-[(2-hydroxyphenyl)ethynyl]benzoic acid (39)
2-Hydroxy-5-(4-methylphenethyl)benzoic acid (40)
2-Hydroxy-5-(3-methoxyphenethyl)benzoic acid (41)
2-Hydroxy-5-(2-methylphenethyl)benzoic acid (42)
5-[(3,4-Dichlorophenyl)ethynyl]-2-hydroxybenzoic acid (43)
5-[(2,4-Dichlorophenyl)ethynyl]-2-hydroxybenzoic acid (44)
5-[(2,4-Dimethylphenyl)ethynyl]-2-hydroxybenzoic acid (45)
5-[(2-Chloro-4-methylphenyl)ethynyl]-2-hydroxybenzoic acid (46)

In further preferred embodiments, the HNF4 modulator of the present invention is based on the structure of compound 4 comprising the structure

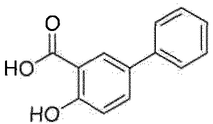

Some optimized HNF4 modulators of the invention are based on compound 4 leading to HNF4 agonists with even lower nanomolar potency and affinity. These properties make the HNF4 modulators of the invention suitable tools to modulate HNF4 activity for in vitro studies or for therapeutic purposes. In further preferred embodiments, compound 4 has been optimized by including sub-structures including, but not limited to variants of the salicylic acid motif, the phenyl substituent position or the biphenyl motif. All active compounds were counter-screened for unspecific effects on the ligand-independent transcriptional inducer Gal4-VP16. The tested compounds demonstrated strong binding affinity, robust HNF4 activation in a cellular setting, favorable selectivity and low cytotoxicity.

Preferred HNF4-modulating compounds that are based on compound 4 and that efficiently modulate HNF4 activity are one or more of the following:

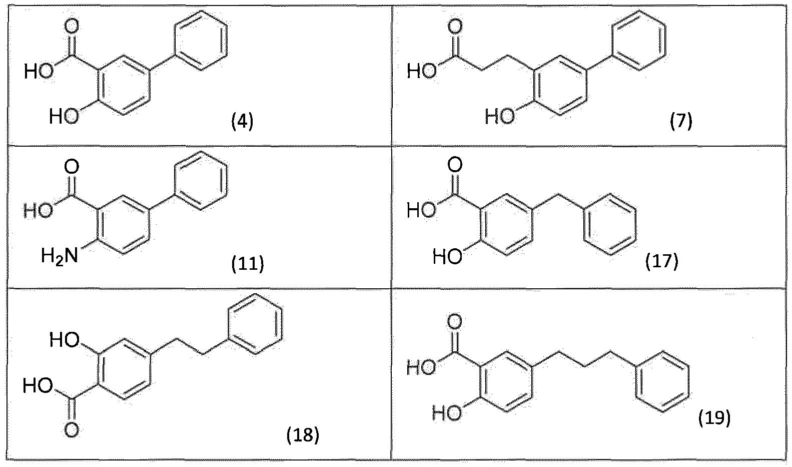

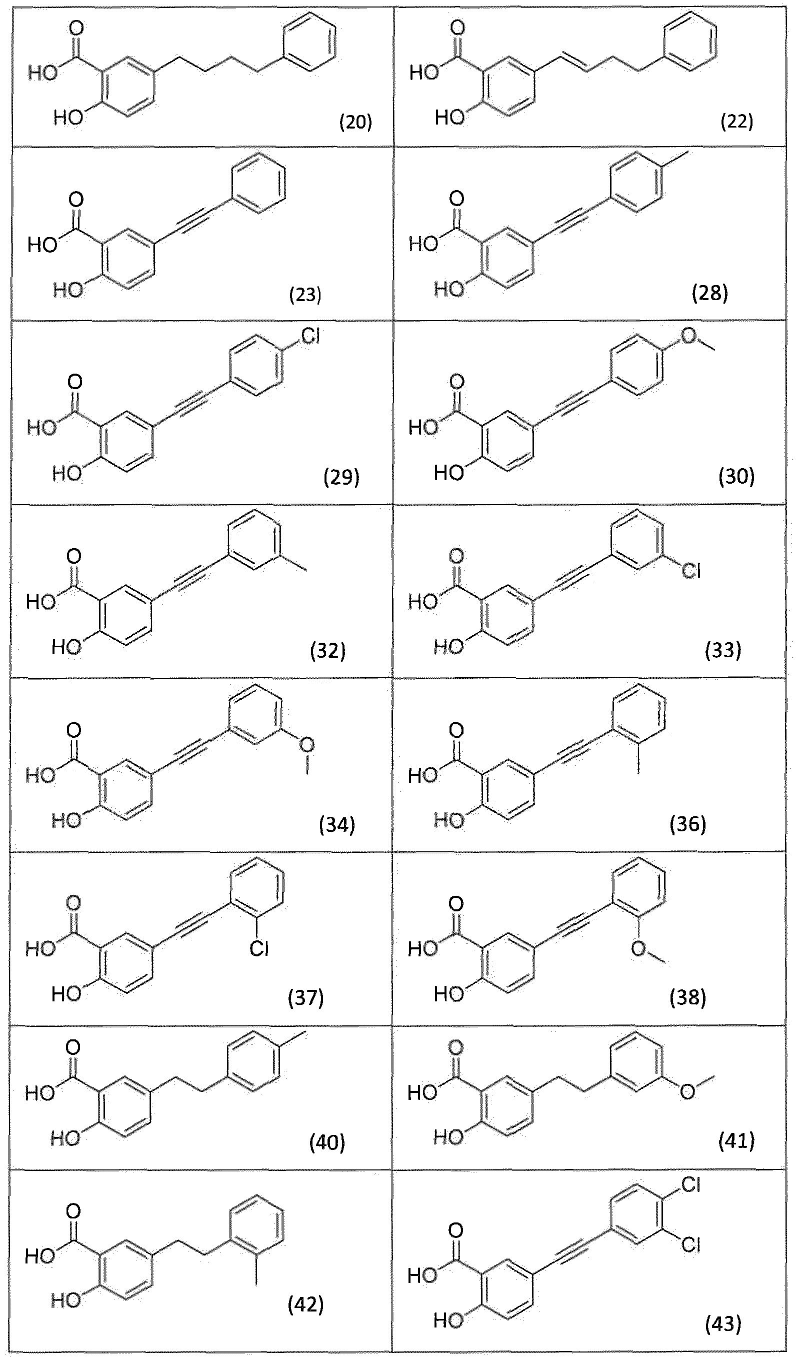

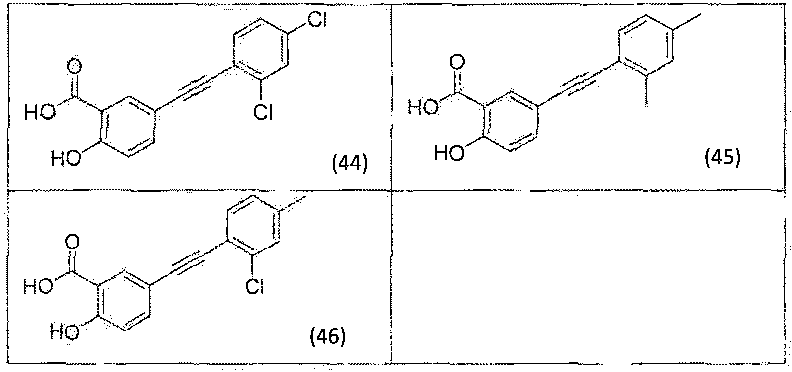

Gal4-hybrid reporter gene assays demonstrated that these compounds exhibit strong potency as indicated by low EC₅₀(HNF4α) values. This is supported by SAR analyses that revealed that bulky groups were tolerated in 5-position of the basic benzene structure.

In more preferred embodiments, the HNF4 modulator of the invention has even lower EC₅₀(HNF4α) values and is therefore selected from the group consisting of
2-Hydroxy-5-(3-phenylpropyl)benzoic acid (19)
2-Hydroxy-5-(4-phenylbutyl)benzoic acid (20)
(E)-2-Hydroxy-5-styrylbenzoic acid (22)
2-Hydroxy-5-(4-methylphenylethynyl)benzoic acid (28)
5-[(4-Chlorophenyl)ethynyl]-2-hydroxybenzoic acid (29)
2-Hydroxy-5-[(4-methoxyphenyl)ethynyl]benzoic acid (30)
2-Hydroxy-5-(3-methylphenylethynyl)benzoic acid (32)
5-[(3-Chlorophenyl)ethynyl]-2-hydroxybenzoic acid (33)
2-Hydroxy-5-[(3-methoxyphenyl)ethynyl}benzoic acid (34)
2-Hydroxy-5-(2-methylphenylethynyl)benzoic acid (36)
5-[(2-Chlorophenyl)ethynyl]-2-hydroxybenzoic acid (37)
2-Hydroxy-5-[(2-methoxyphenyl)ethynyl]benzoic acid (38)
2-Hydroxy-5-(4-methylphenethyl)benzoic acid (40)
2-Hydroxy-5-(3-methoxyphenethyl)benzoic acid (41)
2-Hydroxy-5-(2-methylphenethyl)benzoic acid (42)
5-[(3,4-Dichlorophenyl)ethynyl]-2-hydroxybenzoic acid (43)
5-[(2,4-Dichlorophenyl)ethynyl]-2-hydroxybenzoic acid (44)
5-[(2,4-Dimethylphenyl)ethynyl]-2-hydroxybenzoic acid (45)
5-[(2-Chloro-4-methylphenyl)ethynyl]-2-hydroxybenzoic acid (46).

In particularly preferred embodiments, the HNF4 modulators comprise the formula: 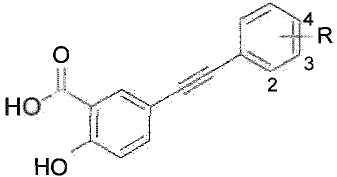 wherein R2 is -H, -CH₃, -Cl, -OCH₃; R3 is -H, -CH₃, -Cl, -OCH₃; R4 is -H -CH₃, -Cl, -CH₃.

In some embodiments, R3 in said formula is Cl and R4 is CI.

In some embodiments, R2 in said formula is Cl and R4 is CI.

In some embodiments, R2 in said formula is CH₃ and R4 is CH₃.

In some embodiments R2 in said formula is Cl and R4 is CH₃.

As shown herein, these compounds offer high HNF4 activation efficiency and are less prone to exhibit side activity on estrogen receptors (ERs).

A preferred HNF4-modulating compound is 5-[(2-Chloro-4-methylphenyl)ethynyl]-2-hydroxybenzoic acid (46) having the structure

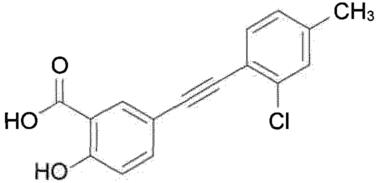

This compound exhibits a strong binding affinity and results in a favorable HNF4 activity, while retaining high selectivity and low cytoxicity.

The present invention also relates to pharmaceutical compositions, comprising an HNF4 modulator as defined herein and a pharmaceutically acceptable carrier or diluent. In some embodiments, the pharmaceutical composition comprises a modulator of HNF4 for the use in the treatment or prevention of a disease that is associated with HNF4 malfunction or genetic mutations of the HNF4 gene. In preferred embodiments, the disease is associated with HNF4α malfunction or genetic mutations of the HNF4α gene. In alternative embodiments, the disease is associated with HNF4γ malfunction or genetic mutations of the HNF4γ gene.

In one aspect, the pharmaceutical composition comprises an HNF4 modulator, as defined herein, and a pharmaceutically acceptable carrier or diluent for the use in the prevention or treatment of type 2 diabetes, maturity-onset diabetes of the young type 1 (MODY1), maturity-onset diabetes of the young type 3 (MODY3), neonatal hyperinsulinemic hypoglycemia, gastrointestinal disease, liver disease, pancreatic disease, gastrointestinal cancer, inflammatory bowel disease (IBD), atherosclerosis, metabolic dysfunction, metabolic syndrome, insulin resistance or lipid disorders. Preferably, the HNF4 modulator in the pharmaceutical composition is an HNF4α agonist.

In a preferred embodiment, the HNF4 modulator in the pharmaceutical composition is provided in a single or multiple dose form. The pharmaceutical composition may be formulated with any known pharmaceutically acceptable carrier or diluent, as well as any other known adjuvants and excipients, in accordance with conventional techniques. The pharmaceutically acceptable carriers, diluents, adjuvants and excipients should be suitable for the chosen modulator of the present invention and the chosen mode of administration. A pharmaceutical composition of the present invention may also include diluents, fillers, salts, buffers, detergents (e.g., a nonionic detergent), stabilizers (e.g., sugars or protein-free amino acids), preservatives, tissue fixatives, solubilizers, and/or other materials suitable for inclusion in a pharmaceutical composition.

The pharmaceutical compositions of the present invention can be formulated by methods known to those skilled in the art. For example, such pharmaceutical compositions can be used parenterally, as injections which are sterile solutions or suspensions including the compositions along with water or another pharmaceutically acceptable liquid. For example, such compositions may be formulated as unit doses that meet the requirements for the preparation of pharmaceuticals by appropriately combining the compositions with pharmaceutically acceptable carriers, diluents, adjuvants or excipients, specifically with sterile water, physiological saline, a vegetable oil, emulsifier, suspension, surfactant, stabilizer, flavoring agent, excipient, vehicle, preservative, binder or such. In such preparations, the amount of active ingredient is adjusted.

The HNF4 modulator of the present invention can be combined in a single composition for use in the treatment or prevention of a disease that is associated with HNF4 malfunction or genetic mutations of the HNF4 gene, in particular type 2 diabetes, maturity-onset diabetes of the young type 1 (MODY1), maturity-onset diabetes of the young type 3 (MODY3), neonatal hyperinsulinemic hypoglycemia, gastrointestinal disease, liver disease, pancreatic disease.

In some embodiments, the dosage form containing the HNF4 modulator is prepared by combining the HNF4 modulator together with a pharmaceutically acceptable carrier, diluent, excipient, or auxiliary. Such a pharmaceutically acceptable agent is compatible with the other ingredients of the formulation and not substantially deleterious to the recipient thereof. Examples of carriers include, but are not limited to, poly(lactic acid) (PLA) microspheres, poly(D,L-lactic-coglycolic- acid) (PLGA) microspheres, liposomes, micelles, inverse micelles, lipid cochleates, and lipid microtubules. A carrier may comprise a buffered salt solution such as PBS, BBS or HBSS.

Also disclosed herein are methods for treating subject by administrating an HNF4 modulator, e.g. an HNF4α agonist. The subject can be treated by administering the HNF4 modulator to the subject or by administering the HNF4 modulator to cells ex vivo prior to introduction of the cells to the subject. The compounds and pharmaceutical compositions described herein can be administered to the subject in a number of ways depending on whether local or systemic treatment is desired, and on the area to be treated.

Moreover, a compound or pharmaceutical composition can be administered to a subject vaginally, rectally, intranasally, orally, by inhalation, or parenterally, for example, by intradermal, subcutaneous, intramuscular, intraperitoneal, intrarectal, intraarterial, intralymphatic, intravenous, intrathecal and intratracheal routes. Parenteral administration, if used, is generally characterized by injection. Injectables can be prepared in conventional forms, either as liquid solutions or suspensions, solid forms suitable for solution or suspension in liquid prior to injection, or as emulsions. A more recently revised approach for parenteral administration involves use of a slow release or sustained release system such that a constant dosage is maintained.

Preparations for parenteral administration include sterile aqueous or non-aqueous solutions, suspensions, and emulsions, which may also contain buffers, diluents and other suitable additives. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives can also be present such as, for example, antimicrobials, anti-oxidants, chelating agents, and inert gases and the like.

In some embodiments, the subject suffering from type 2 diabetes or MODY1 is treated by administering an HNF4 modulator. In some embodiments, the subject may be at risk of developing type 2 diabetes or MODY1. The present disclosure provides methods of treating a subject having type 2 diabetes or MODY1 or at risk of developing type 2 diabetes or MODY1, the methods comprising administering an HNF4 modulator to the subject. Preferably, the HNF4 modulator administered to the subject is an HNF4α agonist based on one of the compounds described herein. In some embodiments, administering an HNF4 modulator to a subject having type 2 diabetes or MODY1 may be carried out to prevent development of a more severe form of type 2 diabetes or MODY1. In any of the embodiments described herein, the methods can be used to improve type 2 diabetes or MODY1.

In some other embodiments, a subject suffering from liver disease or pancreatic disease is treated by administering an HNF4 modulator of the invention. The subject may be at risk of developing liver disease or pancreatic disease. The present disclosure provides methods of treating a subject having liver disease or pancreatic disease, or at risk of developing liver disease or pancreatic disease, the methods comprising administering an HNF4 modulator to the subject. Preferably, the HNF4 modulator administered to the subject is an HNF4α agonist based on one of the compounds described herein. In some embodiments, administering an HNF4 modulator to a subject having liver disease or pancreatic disease may be carried out to prevent development of a more severe form of liver disease or pancreatic disease. In any of the embodiments described herein, the methods can be used to improve liver disease or pancreatic disease.

The invention also concerns in vitro methods of analyzing the effects of hepatocyte nuclear factor 4 (HNF4) activity in a biological system, comprising the modulation of HNF4 activity by inserting an HNF4 modulator of the invention into a cell or a cell culture.

The HNF4 modulators can also be used in methods for identifying compounds that affect HNF4 regulation, the method comprising bringing into contact the HNF4 modulator and an HNF4-regulated gene, and detecting changes in the expression of the HNF4-regulated gene in the presence and absence of a test compound, wherein a difference in expression of the HNF4-regulated gene in the presence of the test compound relative to expression of the HNF4-regulated gene in the absence of the test compound indicates a compound that affects HNF4 regulation. Preferably said HNF4-regulated gene is the HNF4α gene. Alternatively, said HNF4-regulated gene is the HNF4γ gene.

The present invention will be illustrated in the following examples. By no means shall the invention be limited to these specific examples. The invention also covers any modifications, variants, feature combinations or combinations of embodiments described herein. The invention furthermore also covers not only these specific compounds mentioned herein, but also their results or derivatives thereof.

### Brief Description of the Drawings:

- Fig. 1: shows the binding of an exemplary HNF4 modulator to the HNF4α LBD using isothermal titration calorimetry (ITC),
- Fig. 2: shows in vitro profiling of a tested HNF4 agonist,
- Fig. 3: shows the X-ray structure of the HNF4α LBD in complex with an example compound.

### Detailed Description of the Invention:

It is to be understood that the disclosed methods and compositions are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

As shown in the following, compound 4 has been found as antagonist of HNF4 by screening a drug fragment library in cellular Gal4-HNF4α hybrid reporter gene assay. Compound 4 comprises the following generic structure:

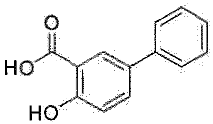

Activity of the compounds was tested in the cellular Gal4-HNF4α hybrid reporter gene assay employing a Gal4-responsive firefly luciferase reporter and a constitutively active renilla luciferase for normalization purposes. All compounds were additionally screened for effects on the ligand-independent transcriptional inducer Gal4-VP16 to exclude potential unspecific activity.

First, the effect of the salicylic acid motif and its potential interaction has been tested. The results are summarized in Table 1.

**Table 1: SAR of the salicylic acid motif**

| **ID** | **structure** | **EC₅₀/IC₅₀(HNF4α) (max./min. act.) ^{a}** |
|---|---|---|
| **4** | 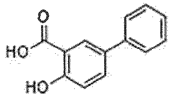 | **IC₅₀ = 8±2 µM (57±4% min.)** |
| **7** | 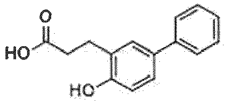 | **IC₅₀ = 16±3 µM (49±4% min.)** |
| **11** | 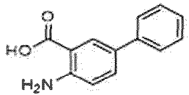 | **IC₅₀ = 100±37 µM (40±15% min.)** |

| | | |
|---|---|---|
| ^{a} HNF4α modulation was determined in a Gal4-hybrid reporter gene assay. Minimum remaining activity (min. act.) is relative to the 0.1% DMSO ctrl. All active compounds were counter-screened for unspecific effects on the ligand-independent transcriptional inducer Gal4-VP16. All data are the meantS.E.M., n≥3. | | |

The activity of the tested compounds is expressed in EC₅₀/IC₅₀ (HNF4α). As can be seen, the extension from the aryl carboxylic acid to an aryl propionic acid, as present in compound 7, was tolerated with slightly reduced potency but enhanced efficacy. Although the anthranilic acid motif of compound 11 retained a somewhat weaker HNF4 modulation, this compound is still suitable for HNF4 modulation.

SAR confirmed that the salicylic acid motif is preferred with importance of both the acidic residue for potential ionic interactions and the hydroxyl group as potential H-bond donor.

Next, the phenyl substituent position was varied based on compound 4. Variants of the compounds bearing the phenyl substituent at different positions have been tested and are shown in Table 2.

**Table 2: Variation of the phenyl substituent position**

| **ID** | structure | EC₅₀/IC₅₀(HNF4α) (max./min. act.) ^{a} |
|---|---|---|
| **4** | 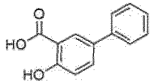 | IC₅₀ = 8±2 µM (57±4% min.) |
| **14** | 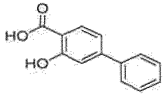 | EC₅₀ = 4±1 µM (152±4% max.) |
| **15** | 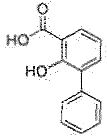 | IC₅₀ = 24±9 µM (47±11% min.) |
| **16** | 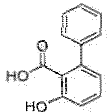 | inactive (100 µM) |

| | | |
|---|---|---|
| ^{a} HNF4α modulation was determined in a Gal4-hybrid reporter gene assay. Maximum activation (max. act.) and minimum remaining activity (min. act.) are relative to the 0.1% DMSO ctrl. All active compounds were counter-screened for unspecific effects on the ligand-independent transcriptional inducer Gal4-VP16. All data are the mean±S.E.M., n≥3. | | |

Moving the phenyl motif from the 5-position of compound 4 to the 4-position in compound 14 interestingly inverted the activity from inverse agonism to HNF4 activation, with considerable potency. The 3-phenyl analogue of compound 15 retained similar inverse agonism as compound 4, while 6-phenyl substitution in compound 16 was not tolerated. This experiment supports the notion that the 5-position, as provided by compound 4, is beneficial for the HNF4 modulators of the invention.

Based on compound 4, bulky hydrophobic groups were further explored. Table 3 summarizes the results of an extension of the biphenyl motif.

**Table 3 - Extension of the biphenyl motif**

| **ID** | **structure** | EC₅₀/IC₅₀(HNF4α) (max./min. act.) ^{a} |
|---|---|---|
| **4** | 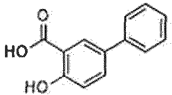 | IC₅₀ = 8±2 µM (57±4% min.) |
| **17** | 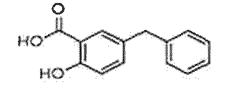 | IC₅₀ = 60±14 µM (50±6% min.) |
| **18** | 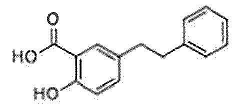 | E C₅₀ = 1.8±0.2 µM (192±5% max.) |
| **19** | 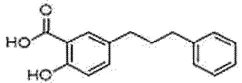 | IC₅₀ = 0.4±0.2 µM (58±3% min.) |
| **20** | 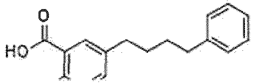 | IC₅₀ = 0.32±0.07 µM (51±2% min.) |
| **22** | 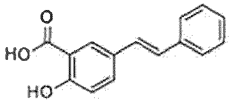 | EC₅₀ = 0.06±0.02 µM (142±2% max.) |
| **23** | 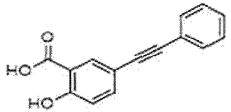 | EC₅₀ = 0.33±0.08 µM (165±5% max.) |

| | | |
|---|---|---|
| ^{a} HNF4α modulation was determined in a Gal4-hybrid reporter gene assay. Maximum activation (max. act.) and minimum remaining activity (min. act.) are relative to the 0.1% DMSO ctrl. All active compounds were counter-screened for unspecific effects on the ligand-independent transcriptional inducer Gal4-VP16. All data are the meantS.E.M., n≥3. | | |

A homologization of compound 4 to the benzyl salicylate of compound 17 was not favored but further extension to the phenethylsalicylic acid analogue in compound 18 enhanced potency by around 5-fold and interestingly inverted the activity profile from inverse agonism to agonism. The corresponding phenylpropyl motif of compound 19 and phenylbutyl motif of compound 20 also displayed significantly enhanced potency but returned to inverse agonist activity. These results suggested that the phenylethylsalicylate in compound 18 forms a more optimized complex with the receptor, thereby stabilizing the active state. Based on these results, the 5-substituted scaffold has proven to be the most promising scaffold for the development of HNF4 modulators. Despite lower potency, compound 23 offered stronger HNF4 activation efficacy and appeared less prone to exhibit side activity on estrogen receptors (ERs) compared to compound 22. Therefore, compound 23 was selected for further optimization to analyze the impact of substituents on the terminal phenyl motif. The results are summarized in Table 4.

**Table 4 - Substituent SAR on the terminal phenyl motif**

| **ID** | 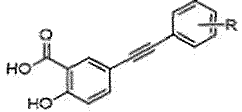 R= | EC₅₀(HNF4α) (max. act.) ^{a} |
|---|---|---|
| **23** | - | 0.33±0.08 µM (165±5%) |
| **28** | 4-CH₃ | 0.014±0.005 µM (175±7%) |
| **29** | 4-Cl | 0.04±0.02 µM (165±4%) |
| **30** | 4-OCH₃ | 0.13±0.03 µM (151±4%) |
| **32** | 3-CH₃ | 0.15±0.04 µM (156±5%) |
| **33** | 3-Cl | 0.25±0.07 µM (129±3%) |
| **34** | 3-OCH₃ | 0.06±0.02 µM (147±6%) |
| **36** | 2-CH₃ | 0.05±0.01 µM (160±3%) |
| **37** | 2-Cl | 0.013±0.003 µM (168±3%) |
| **38** | 2-OCH₃ | 0.041±0.006 µM (187±4%) |

| | | |
|---|---|---|
| ^{a} HNF4α modulation was determined in a Gal4-hybrid reporter gene assay. Maximum activation (max. act.) and minimum remaining activity (min. act.) are relative to the 0.1% DMSO ctrl. All active compounds were counter-screened for unspecific effects on the ligand-independent transcriptional inducer Gal4-VP16. All data are the meantS.E.M., n≥3. | | |

Substituents were introduced with various steric and electrostatic characteristics in all positions of the terminal phenyl ring to determine the optimal group at each site. In 4-position, compound 28 comprising a methyl group, and compound 29 comprising chlorine were highly favored and boosted HNF4α agonist potency. A methoxy motif, as found in compound 30, was tolerated. Similarly, in 3-position, a preference for methoxy (compound 34) over methyl (compound 32) and chlorine (compound 33) was observed. In 2-position, methyl (compound 36), chlorine (compound 37) and methoxy (compound 38) were all highly favored. These results on the substituent SAR of the terminal ring suggested that hydrophobic groups were strongly preferred in all positions, while any polar modification was less tolerated. Inductive effects seemed of little importance, as methyl and chloro substituents displayed similar effects on potency in all positions. Based on the remarkable HNF4α agonist potencies of compound 28 and compound 37 and their superior efficacies, modifications in 4- and 2-positions emerged to be the most promising.

It was also found that compounds bearing a hydroxyl group in the -2, -3 or -4 position, i.e. in which R = 2-OH, 3-OH or 4-OH are less potent as HNF4 agonists.

Next, it was investigated whether the most favored substituents discovered for the diphenylethyne series were indeed tolerated on the saturated analogues and provided slight improvements in potency and efficacy over the unsubstituted compound 18. As shown in Table 5, the diphenylethyne derivatives outmatched compounds 40-42 by two orders of magnitude.

**Table 5 - SAR transfer to the saturated scaffold**

| **ID** | 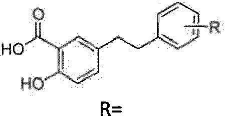 | EC₅₀(HNF4α) (max. act.) ^{a} |
|---|---|---|
| **18** | - | 1.8±0.2 µM (192±5%) |
| **40** | 4-CH₃ | 1.2±0.2 µM (212±12%) |
| **41** | 3-OCH₃ | 3.4±0.6 µM (210±11%) |
| **42** | 2-CH₃ | 1.3±0.2 µM (234±11%) |

| | | |
|---|---|---|
| ^{a} HNF4α modulation was determined in a Gal4-hybrid reporter gene assay. Maximum activation (max. act.) and minimum remaining activity (min. act.) are relative to the 0.1% DMSO ctrl. All active compounds were counter-screened for unspecific effects on the ligand-independent transcriptional inducer Gal4-VP16. All data are the mean±S.E.M., n≥3. | | |

To further improve the compounds, the rigid diphenylethyne scaffold was investigated and double substitution as well as combinations of preferred substituents were tested. The results are summarized in Table 6.

**Table 6 - Double substitution and SAR fusion**

| **ID** | 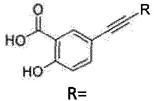 | EC₅₀(HNF4α) (max. act.) ^{a} |
|---|---|---|
| **43** | 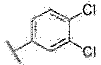 | 0.3±0.1 µM (188±11%) |
| **44** | 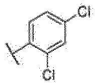 | 0.03±0.01 µM (192±9%) |
| **45** | 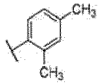 | 0.03±0.01 µM (177±5%) |
| **46** | 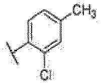 | 0.006±0.003 µM (192±6%) |

| | | |
|---|---|---|
| ^{a} HNF4α modulation was determined in a Gal4-hybrid reporter gene assay. Maximum activation (max. act.) and minimum remaining activity (min. act.) are relative to the 0.1% DMSO ctrl. All active compounds were counter-screened for unspecific effects on the ligand-independent transcriptional inducer Gal4-VP16. All data are the mean±S.E.M., n≥3. | | |

As shown in Table 6, the 3,4-dichloro substitution in compound 43 and 2,4-dichloro substitution in compound 44 strongly supported the preference for a 2,4-disubstitution, showing a 10-fold higher potency of compound 44. The 2,4-dichloro substitution in compound 44 and 2,4-dimethyl substitution in compound 45 were equipotent, suggesting that hydrophobic substituents are driving potency rather than inductive effects. As methyl was most favored in 4-position (compound 28) and chlorine emerged as preferred 2-substituent (compound 37), these modifications were combined in compound 46, which displayed remarkable HNF4α agonist potency (EC₅₀ = 6 nM).

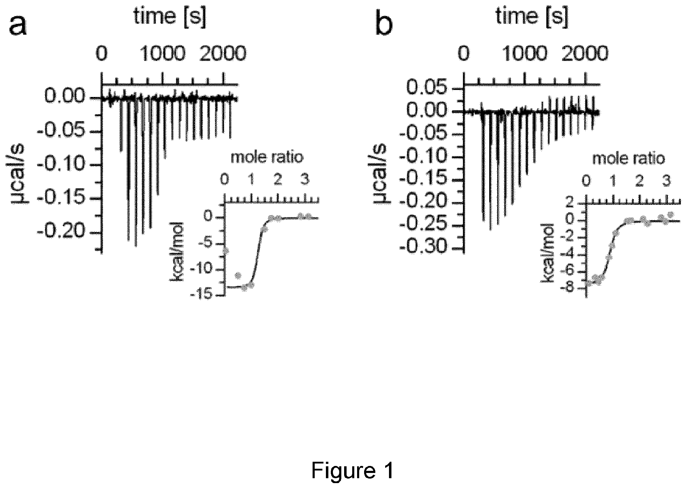

As shown in Figure 1, the binding of compound 46 to the HNF4α LBD was confirmed by isothermal titration calorimetry (ITC). (a) Compound 46 displayed high affinity to the lipid-free HNF4α LBD (K_{d} = 23 nM). (b) Presence of a fatty acid bound to the orthosteric ligand binding site diminished affinity of compound 46 in ITC (apparent K_{d} = 0.36 µM), pointing to competitive binding to the orthosteric pocket.

Furthermore, strong affinity of compound 46 to the HNF4α LBD could be shown by isothermal titration calorimetry (ITC). HNF4α typically contains a residual fatty acid bound to the orthosteric site, which is also found in recombinantly expressed protein. When this lipid was removed before ITC experiments, compound 46 bound to the HNF4α LBD with a K_{d} value of 23 nM (Figure 1a). Presence of the lipid diminished affinity of compound 46 (K_{d} = 0.36 µM, Figure 1b) indicating that the ligand likely bound to the orthosteric site in a competitive manner.

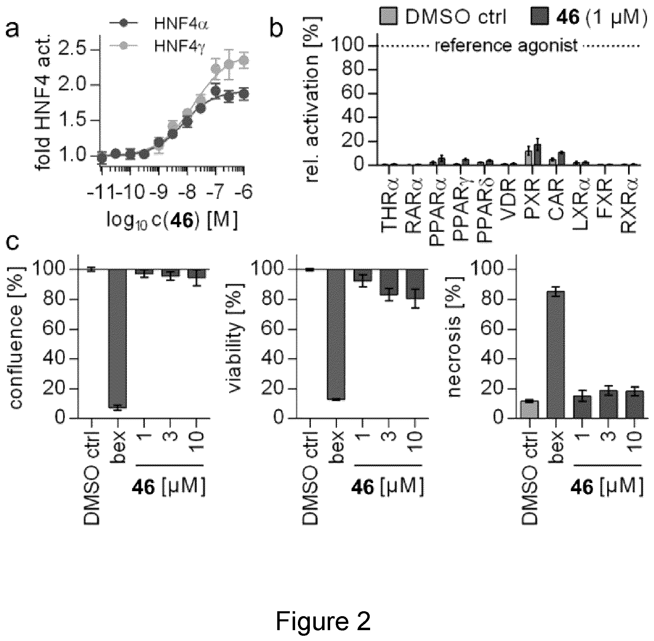

Compound 46 has been proven to be a highly efficient high-affinity HNF4 agonist. This compound is particularly suitable for studying the biology and therapeutic potential of HNF4. To validate the suitability of compound 46 as a chemical tool for in vitro studies, the compound was profiled for selectivity and toxicity (Figure 2).

Figure 2 shows the in vitro profiling of the HNF4 agonist compound 46. (a) Compound 46 activated HNF4α (EC₅₀ 6±3 nM) and HNF4γ (EC₅₀ 17±7 nM) with low nanomolar potency. Data (meantS.E.M.) from Gal4-hybrid reporter gene assays (n≥3). (b) Compound 46 (1 µM) displayed favorable selectivity for HNF4 over other lipid-activated and promiscuous nuclear receptors. Data (mean±SD) from uniform Gal4-hybrid reporter gene assays (n≥3). (c) Compound 46 (up to 10 µM) was non-toxic in a multiplex toxicity assay monitoring proliferation (confluence), cell viability (metabolic activity, WST-8) and necrosis (NucFix Red) in HepG2 cells. Data are the mean±SD, n=4.

Expectedly, compound 46 exhibited similar agonism on the highly related HNF4α and HNF4γ isoforms in cellular assays (Figure 2a, Table 7) but displayed favorable selectivity over other fatty acid/lipid-sensing (THR, RAR, PPAR, VDR, LXR, FXR, RXR) and promiscuous (CAR, PXR) nuclear receptors, with no activation >20% of the reference agonist detectable at 1 µM (Figure 2b). A multiplex toxicity assay in HepG2 cells, monitoring proliferation (confluence), viability (WST-8 reagent), and necrosis (NucFix Red) detected no toxicity of compound 46 up to 10 µM (Figure 2c). These characteristics thus supported suitability of compound 46 to serve as chemical tool to study HNF4 biology in vitro.

**Table 7 - Characteristics of the HNF4 agonist compound 46**

| | 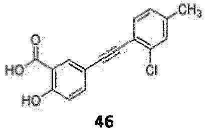 |
|---|---|
| EC₅₀(HNF4α) (max. act.) ^{a} | 6±3 nM (192±6%) |
| EC₅₀(HNF4γ) (max. act.) ^{a} | 17±7 nM (246±16%) |
| K_{d} (HNF4α LBD) ^{b} | 23±5 nM (lipid-free) |
| cytotoxicity | > 10 µM |
| logP ^{c} | 4.07 |

| | |
|---|---|
| ^{a} EC₅₀ and max. act. (vs. DMSO) from Gal4-hybrid reporter gene assay. ^{b} K_{d} from isothermal titration calorimetry (ITC). ^{c} logP was predicted with ALOGPS2.1 | |

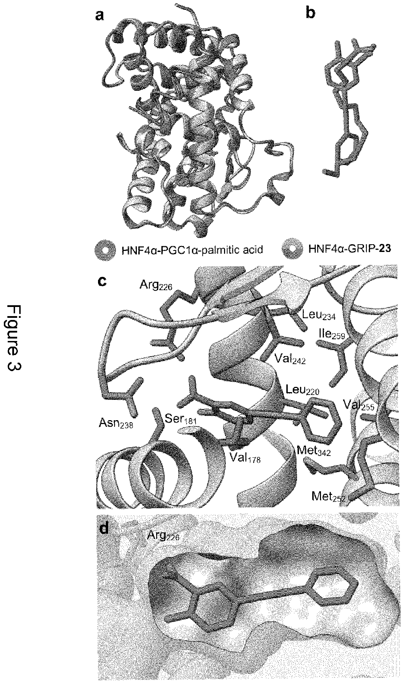

To rationalize and fully validate HNF4 modulation by the new phenylethynylsalicylic acid scaffold, structural insights were gained by successfully co-crystallizing the HNF4α LBD in complex with the unsubstituted representative compound 23 (pdb ID 9ibr; Figure 3).

Figure 3 shows the X-ray structure (pdb ID 9ibr) of the HNF4α LBD in complex with compound 23 and a fragment of the co-activator GRIP. (a) Overview of the HNF4α LBD complex with compound 23, superimposed with the myristic acid-bound receptor (pdb ID 3fs1²³). (b) Compound 23 and the natural ligand palmitic acid display highly similar binding modes to the HNF4α LBD. The binding modes extracted from the aligned co-crystal structures are shown. (c,d) Binding mode of compound 23 in the HNF4α ligand binding site, with pocket-forming residues (c) and pocket surface (d).

The asymmetric unit consisted of a protein monomer in complex with a fragment of the coactivator GRIP and resembled the previously reported structure of HNF4α in complex with myristic acid (pdb ID 3fs1²³, Figure 3a). The canonical homodimer with perfect 2-fold symmetry was observed in the crystal packing interactions. Compound 23 is bound to the orthosteric pocket between helices 3, 6, 8 and 11, which is also occupied by the natural ligand myristic acid (Figure 3a, b). While the salicylic acid head engaged in a polar interaction network with Ser181 and Arg226, the remainder of the molecule was bound between hydrophobic residues lining the binding pocket (Figure 3c).

The X-ray structure also rationalized the observed SAR. The rigid linear geometry of compound 23 matched very well with the pocket characteristics (Figure 3d) explaining the superior potency of the alkyne in compound 23 over the saturated analogue compound 18 and the loss of activity with the alternative regiochemistry of compound 26. Additionally, the limited space available in para-position of the carboxylic acid aligned with lack of HNF4 agonism of the para-methyl derivative of compound 27 while the pocket around the terminal phenyl motif was more ample and could accommodate additional substituents.

In summary, the HNF4 modulators produced by the present invention show a strong binding affinity, robust HNF4 activation in a cellular setting, favorable selectivity and low cytotoxicity. The X-ray structure of the HNF4α complex with compound 23 validates direct modulation by the new ligand scaffold and provides deeper molecular insights. The compounds of the invention have proven to be potent HNF4 modulators and as such are valuable therapeutic tools for use in the treatment of diseases that are associated with HNF4 malfunction or genetic mutations of the HNF4 gene.

### Material and Methods:

### Synthetic procedures and analytical characterization data

All used chemicals and solvents were purchased from commercial sources (BLDpharm, Alfa Aesar, Sigma-Aldrich, Thermo Scientific, Acros Organics, Honeywell Fluka, TCI, abcr, Chempur and Chemsolute) of reagent grade (≥ 95 % pure) and used without further purification. Compounds 6, 11, 15, 17-18 and 23 were obtained from Enamine Ltd., compounds 7, 9, 12, 14 and 21 were purchased from TRC and compound 10 was acquired from BLDpharm. All reactions were carried out in oven dried glassware and in absolute solvents. If required, reactions were carried out with argon as inert gas. Monitoring of the reactions by thin-layer chromatography (TLC) was performed using silica-coated (particle size of 60 µm) aluminum plates (ALUGRAM^{®} Xtra SIL G/UV254 0.2 mm, Machery-Nagel) with detection under UV-light (254 nm and 366 nm). Solvents used for compound purification (n-hexane, cyclohexane, EtOAc, MeOH and H₂O) were of technical grade and used without further purification. Preparative manual normal phase column chromatography (CC) was carried out using silica 60 from Macherey-Nagel. Purification by automated CC was performed using a puriFlash XS520Plus system (Advion Interchim Scientific, Monrlucon, France) with high performance spherical silica columns (normal phase, PF-30SIHP-F0012, - F0025 or -F0040) with a gradient of n-hexane/cyclohexane containing 0.1 % acetic acid to EtOAc containing 0.1 % acetic acid (auto NP-CC) or C18HP columns (reversed phase, PF-30C18HP-F0012, -F0025, -F0040) with a gradient of H₂O containing 0.1 % acetic acid to MeOH/MeCN containing 0.1 % acetic acid (auto RP-CC). For nuclear magnetic resonance (NMR) spectroscopy of intermediate products Bruker AV300, AV400 or AV400HD spectrometers (Bruker Corporation, Billerica, MA, USA) were used. NMR spectra of final compounds were recorded on a Bruker AV500HD spectrometer. Chemical shifts (δ) are reported in ppm relative to residual proton or carbon signals in the NMR solvent²⁷ and approximate coupling constants (J) are shown in hertz (Hz). NMR spectra were processed and analyzed with MestReNova (version 12.0.1-20560 Mestrelab Research S.L., Santiago de Compostela, A Coruña, Spain). Electrospray ionization mass spectra (ESI-MS) were obtained on a VG Platform II (Thermo Fischer Scientific, Inc., Waltham, MA, USA). High-resolution mass spectra (HRMS) were obtained with a Thermo Finnigan LTQ FT instrument for electron impact ionization (EI) or electrospray ionization (ESI). The purity of all final compounds was analyzed with high performance liquid chromatography (HPLC) on a Hitachi Chromaster (Hitachi Ltd., Tokyo, Japan) with a 5160 pump system, using a 5260 Autosampler and a 5430 Diode Array Detector (DAD) equipped with an ACE^{®} C18 5 µM 250 x 4 mm column (Avantor, Inc., Radnor, Pennsylvania, USA). For UV-detection at 245 nm and 280 nm a gradient (H₂O + 0.1% formic acid / MeCN 80:20 isocratic for 5 minutes to 95% MeCN after 25 minutes and 95% MeCN for additional 5 minutes) at a flow rate of 1 mL/min was used. All final compounds for biological evaluation had a purity of at least 95 % according to HPLC-UV analysis at 245 nm and 280 nm.

**General procedure (a)** - **Suzuki reaction.** The respective bromobenzoic acid (1.0 eq.) and Na₂CO₃ (3.0 eq.) were weighed into a two necked flask pre-charged with argon and dissolved in a degassed 4:1 mixture of 1,4-dioxane and H₂O (c = 0.5 M). Phenylboronic acid (2.0 eq.) and a catalytic amount of Pd(PPh₃)₄ (0.05 eq.) were added and the mixture was heated to reflux under argon for 2-4 h. After cooling to room temperature, CH₂Cl₂ (25 mL) was added, and the mixture was extracted with an aqueous NaOH solution (2 M; 4 x 25 mL). The combined aqueous layers were washed with CH₂Cl₂ (2 x 25 mL) and then acidified with concentrated hydrochloric acid (37 %) to pH 1. The aqueous layer was subsequently extracted with EtOAc (4 x 25 mL) and the combined organic layers were dried over Na₂SO₄, filtrated and concentrated under reduced pressure. The resulting residue was purified by automated CC. Compounds 13 and 51-52 were prepared according to this general procedure in 33-60 % yield.

**General procedure (b)** - **Heck reaction.** The respective bromohydroxybenzoic acid (1.0 eq.) and K₂CO₃ (2.0 eq.) were weighed into a two-necked flask pre-charged with argon and dissolved in dry and degassed N,N-dimethylformamide (c = 0.1 M). After a couple of minutes the respective phenylalkene (1.5 eq.), a catalytical amount of Pd(OAc)₂ (0.1 eq.) and pentane-2,4-dione (0.2 eq.) were added and the mixture was heated to 130 °C under argon for 2-4 h. After cooling to room temperature, aqueous hydrochloric acid (10 %; 30 mL) was added and the mixture extracted with EtOAc (4 x 30 mL). The combined organic layers were dried over Na₂SO₄, filtrated and the solvent was evaporated under reduced pressure. The crude product was purified by automated reversed phase CC. Compounds 22, 25 and 58-59 were prepared according to this general procedure in 64-88 % yield.

**General procedure (c)** - **Sonogashira reaction.** The respective bromohydroxybenzoic acid (1.0 eq.) was weighed into a two-necked flask pre-charged with argon and dissolved in piperidine (c = 0.3 M). After the bromohydroxybenzoic acid had been dissolved the respective phenylethynyl (2.0 eq.) was added and the mixture was stirred for a couple of minutes until a catalytical amount of PdCl₂(PPh₃)₂ (0.04 eq.) was added. The resulting mixture was heated to 110 °C under argon for 1-22 h. After cooling to room temperature, CH₂Cl₂ (25 mL) was added, and the mixture was extracted with an aqueous NaOH solution (2 M; 4 x 25 mL). The combined aqueous layers were washed with CH₂Cl₂ (2 x 25 mL), then acidified with concentrated hydrochloric acid (37 %) to pH 1 and subsequently extracted with EtOAc (4 x 25 mL). The combined organic layers were dried over Na₂SO₄, filtrated and the solvent was evaporated under reduced pressure. The crude product was purified by auto RP-CC. Compounds 26-30, 32-34, 36-38 and 44-46 were prepared according to this general procedure in 10-81 % yield.

**General procedure (d)** - **Hydrogenation with Pd/C.** The respective phenylethynyl (1.0 eq.) or styryl (1.0 eq.) was dissolved in MeOH (100 mL) and filled into a 250 mL two-necked flask pre-charged with argon. A catalytic amount of Pd on carbon (10 %; 0.1 eq.) was added to the reaction solution. The mixture was not stirred before it was flushed with hydrogen. The resulting suspension was hydrogenated under ambient pressure at room temperature for 1-3 h at high stirring speed (900 rpm) using a balloon as hydrogen reservoir. Subsequently the reaction suspension was filtered through Celite^{®} 535 which was washed several times with MeOH and the solvent evaporated under reduced pressure. Aqueous hydrochloric acid (10 %; 20 mL) was added to the resulting residue, and the mixture was extracted with EtOAc (4 x 25 mL). The combined organic layers were dried over Na₂SO₄, filtrated and the solvent was evaporated under reduced pressure. Compounds 19-20, 24, 40-42 were prepared according to this general procedure in 65-100 % yield.

**General procedure (e)** - **Demethylation with boron tribromide.** The respective compound containing a methoxy group (1.0 eq.) was placed in a 25 mL two-necked flask pre-charged with argon and suspended in CH₂Cl₂ (c = 0.1 M). The resulting suspension was cooled to -12 °C before BBr₃ (1.0 eq. per oxygen in the molecule) was added dropwise to the reaction suspension. The reaction was stirred for 1-24 h, started at -12°C and allowed to warm to room temperature during the reaction under argon atmosphere. Subsequently the reaction was quenched by the addition of aqueous hydrochloric acid (10 %; 25 mL). The resulting aqueous layer was extracted with EtOAc (4 x 25 mL) and the combined organic layers were dried over Na₂SO₄, filtrated and concentrated under reduced pressure. The crude product was purified by manual or automated CC. Compounds 16, 31, 35 and 39 were prepared according to this general procedure in 16-72 % yield.

**General procedure (f)** - **Synthesis of alkynes from aldehydes.** To a solution of the respective benzaldehyde (1.0 eq.) and anhydrous K₂CO₃ (2.0 eq.) in dry MeOH (c = 0.1 M), dimethyl-1-diazo-2-oxopropylphosphonate (compound 62; 2.0 eq.) was added dropwise. The reaction was stirred overnight at room temperature. By addition of aqueous hydrochloric acid (10%; 25 mL) the reaction was quenched. The aqueous layer was extracted with EtOAc (4 x 25 mL). The combined organic layers were dried over Na₂SO₄, filtrated and the solvent was evaporated under reduced pressure. The crude product was used without further purification. Compounds 63-64 were prepared according to this general procedure in 27-74 % yield.

### 4-(Hydroxymethyl)[1,1'-biphenyl]-3-carboxylic acid (compound 8).

6-Phenylisobenzofuran-1(3*H*)-one (compound 51, 1.0 eq., 80 mg, 0.38 mmol) was dissolved in tetrahydrofuran (5 mL). LiOH·H₂O (20.0 eq., 320 mg, 7.61 mmol) was dissolved in H₂O (2 mL). Subsequently, both solutions were combined and stirred at 100°C under reflux for 6 h. After cooling to room temperature H₂O was added to the reaction solution and the resulting mixture was extracted with CH₂Cl₂ (50 mL). The obtained organic layer was extracted with an aqueous NaOH solution (2 M; 4 x 25 mL). The combined aqueous layers were acidified with concentrated hydrochloric acid (37 %) to pH 1 and extracted four times with EtOAc (25 mL). The combined organic layers were dried over Na₂SO₄ and filtered, and the solvent was evaporated under reduced pressure. The crude product was purified by auto RP-CC (H₂O + 0.1 % AcOH to MeOH + 0.1 % AcOH = 30 % → 95 %). Yield: 37 %. Colorless solid. ¹H NMR (500 MHz, Acetone-*d₆*) δ 8.28 (d, *J =* 2.0 Hz, 1H), 7.89 (dd, *J* = 8.0, 2.0 Hz, 1H), 7.84 (d, *J =* 8.1 Hz, 1H), 7.71 (d, *J* = 7.2 Hz, 2H), 7.50 (t, *J* = 7.7 Hz, 2H), 7.40 (t, *J* = 7.4 Hz, 1H), 4.99 (s, 2H). ¹³C NMR (126 MHz, Acetone-*d₆*) δ 168.80, 144.47, 140.68, 140.38, 131.52, 129.98, 129.88, 129.64, 129.40, 128.55, 127.69, 63.22. MS (ESI⁻): *m*/*z* 227.28 ([M-H]⁻) HRMS (ESI-): *m*/*z* calcd for C₁₄H₁₂O₃-H⁺: 227.0714, found: 227.0714 ([M-H]-).

**6-Hydroxy[1,1'-biphenyl]-3-carboxylic acid (compound 13).** Preparation according to general procedure **(a)** using 3-bromo-4-hydoxybenzoic acid (compound 48, 306 mg, 1.38 mmol) and phenylboronic acid (compound 50, 355 mg, 2.76 mmol) for 4 h. The product was purified by auto NP-CC (n-hexane + 0.1 % AcOH to EtOAc + 0.1 % AcOH = 1.25 % → 10 %). Yield: 33 %. Colorless solid. ¹H NMR (500 MHz, Acetone-*d₆*) δ 9.24 (bs, 1H), 8.03 (d, *J = 2.2* Hz, 1H), 7.92 (dd, *J* = 8.5, 2.2 Hz, 1H), 7.65 - 7.59 (m, 2H), 7.43 (t, *J* = 7.7 Hz, 2H), 7.36 - 7.31 (m, 1H), 7.11 (d, *J* = 8.5 Hz, 1H). ¹³C NMR (126 MHz, Acetone-*d₆*) δ 167.95, 159.39, 138.68, 133.54, 131.50, 130.09, 129.33, 128.97, 127.97, 123.05, 116.85. MS (ESI⁻): *m*/*z* 213.31 ([M-H]⁻). HRMS (ESI⁻): *m*/*z* calcd for C₁₃H₁₀O₃-H⁺: 213.0557, found: 213.0558 ([M-H]⁻).

**3-Hydroxy[1,1'-biphenyl]-2-carboxylic acid (compound 16).** Preparation according to general procedure **(e)** using compound 52 (0.17 g, 0.75 mmol) and BBr₃ (220 µL, 2.26 mmol) for 24 h. The product was purified by manual CC (n-hexane/EtOAc + 1 % AcOH = 4:1). Yield: 72 %. Pale yellow solid. ¹H NMR (500 MHz, Acetone-*d₆*) δ 7.45 (t, *J* = 8.1 Hz, 1H), 7.40 - 7.29 (m, 5H), 6.98 (dd, *J* = 7.5, 0.8 Hz, 1H), 6.80 (dd, *J* = 7.5, 0.8 Hz, 1H). ¹³C NMR (126 MHz, Acetone-*d₆*) δ 172.69, 161.88, 145.64, 143.41, 134.05, 129.17, 128.52, 127.72, 123.10, 116.98, 114.25. MS (ESI⁻): *m*/*z* 213.08 ([M-H]⁻). HRMS (ESI⁻): *m*/*z* calcd for C₁₃H₁₀O₃-H⁺: 213.0557, found: 213.0557 ([M-H]⁻).

**2-Hydroxy-5-(3-phenylpropyl)benzoic acid (compound 19).** For the synthesis of compound 19, (*E*)-2-hydroxy-5-(3-phenylprop-1-en-1-yl)benzoic acid (compound 58) was first prepared according to general procedure **(b)** using 5-bromo-2-hydroxybenzoic acid (compound 53, 324 mg, 1.47 mmol) and allylbenzene (compound 55, 297 µL, 2.20 mmol) for 2 h. Yield: ~81 %. Beige solid. MS (ESI⁻): *m*/*z* 253.31 ([M-H]⁻). Compound 58 (0.17 g, 0.65 mmol) was used without further purification for 3 h to prepare compound 19 according to general procedure **(d).** The product was purified by auto RP-CC (H₂O + 0.1 % AcOH to MeOH + 0.1 % AcOH = 30 % → 95 %). Yield: 65 %. Colorless solid. ¹H NMR (500 MHz, Acetone-*d₆*) δ 10.92 (bs, 1H), 7.73 (d, *J =* 2.2 Hz, 1H), 7.38 (dd, *J* = 8.5, 2.3 Hz, 1H), 7.29 - 7.11 (m, 5H), 6.89 (d, *J* = 8.5 Hz, 1H), 2.68 - 2.58 (m, 4H), 1.96 - 1.88 (m, 2H). ¹³C NMR (126 MHz, Acetone-*d₆*) δ 172.71, 161.26, 143.07, 137.05, 133.79, 130.46, 129.21, 129.12, 126.54, 118.03, 112.78, 35.92, 34.95, 34.12. MS (ESI⁻): *m*/*z* 255.27 ([M-H]⁻). HRMS (ESI⁻): *m*/*z* calcd for C₁₆H₁₆O₃-H⁺: 255.1027, found: 255.1022 ([M-H]⁻).

**2-Hydroxy-5-(4-phenylbutyl)benzoic acid (compound 20).** Preparation according to general procedure **(d)** using compound 59 (0.11 g, 0.41 mmol) for 2 h. Yield: 98 %. Colorless solid. ¹H NMR (500 MHz, Acetone-*d₆*) δ 10.94 (bs, 1H), 7.71 (d, *J* = 2.3 Hz, 1H), 7.36 (dd, *J =* 8.5, 2.3 Hz, 1H), 7.25 (t, *J* = 7.5 Hz, 2H), 7.21 - 7.12 (m, 3H), 6.87 (d, *J =* 8.5 Hz, 1H), 2.68 - 2.57 (m, 4H), 1.70 - 1.58 (m, 4H). ¹³C NMR (126 MHz, Acetone-*d₆*) δ 172.71, 161.22, 143.35, 137.04, 134.04, 130.40, 129.17, 129.06, 126.44, 117.96, 112.74, 36.26, 35.16, 31.92, 31.75. MS (ESI⁻): *m*/*z* 269.30 ([M-H]⁻). HRMS (ESI⁻): *m*/*z* calcd for C₁₇H₁₈O₃-H⁺: 269.1183, found: 269.1168 ([M-H]⁻).

**(*E*)-2-Hydroxy-5-styrylbenzoic acid (compound 22).** Preparation according to general procedure **(b)** using 5-bromo-2-hydroxybenzoic acid (compound 53, 510 mg, 2.30 mmol) and styrene (compound 57, 401 µL, 3.46 mmol) for 3 h. The product was purified by auto RP-CC (H₂O + 0.1 % AcOH to MeCN + 0.1 % AcOH = 5 % → 95 %). Yield: 85 %. Pale yellow solid. ¹H NMR (500 MHz, Acetone-*d₆*) δ 8.07 (d, *J* = 2.3 Hz, 1H), 7.84 (dd, *J* = 8.7, 2.3 Hz, 1H), 7.60 (d, *J =* 7.6 Hz, 2H), 7.36 (t, *J =* 7.7 Hz, 2H), 7.28 - 7.22 (m, 2H), 7.17 (d, *J* = 16.4 Hz, 1H), 6.99 (d, *J* = 8.7 Hz, 1H). ¹³C NMR (126 MHz, Acetone-*d₆*) δ 172.61, 162.58, 138.43, 134.31, 129.92, 129.51, 129.47, 128.26, 128.17, 128.11, 127.23, 118.63, 113.27. MS (ESI⁺): *m*/*z* 263.16 ([M+Na]⁺). HRMS (ESI⁻): *m*/*z* calcd for C₁₅H₁₂O₃-H⁺: 239.0714, found: 239.0713 ([M-H]⁻).

**2-Hydroxy-4-(2-phenylethyl)benzoic acid (compound 24).** Preparation according to general procedure **(d)** using compound 25 (0.10 g, 0.42 mmol) for 2 h. Yield: 99 %. Pale brown solid. ¹H NMR (500 MHz, CD₂Cl₂) δ 10.79 (bs, 1H), 10.24 (s, 1H), 7.75 (d, *J* = 2.2 Hz, 1H), 7.36 (dd, *J* = 8.5, 2.2 Hz, 1H), 7.29 (t, *J =* 7.5 Hz, 2H), 7.20 (t, *J* = 8.4 Hz, 3H), 6.94 (d, *J* = 8.5 Hz, 1H), 2.96 - 2.87 (m, 4H). ¹³C NMR (126 MHz, CD₂Cl₂) δ 175.43, 161.04, 141.98, 138.09, 133.59, 130.68, 129.06, 128.89, 126.53, 118.13, 111.55, 38.28, 37.25. MS (ESI⁻): *m*/*z* 241.12 ([M-H]⁻). HRMS (ESI⁻): *m*/*z* calcd for C₁₅H₁₄O₃-H⁺: 241.0870, found: 241.0867 ([M-H]⁻).

**(*E*)-2-Hydroxy-4-styrylbenzoic acid (compound 25).** Preparation according to general procedure **(b)** using 4-bromo-2-hydroxybenzoic acid (compound 54, 510 mg, 2.30 mmol) and styrene (compound 57, 401 µL, 3.46 mmol) for 2 h. The product was purified by auto RP-CC (H₂O + 0.1 % AcOH to MeCN + 0.1 % AcOH = 5 % → 95 %). Yield: 88 %. Pale brown solid. ¹H NMR (500 MHz, DMSO-*d₆*) δ 7.78 (d, *J* = 8.1 Hz, 1H), 7.63 (d, *J* = 7.4 Hz, 2H), 7.43 - 7.36 (m, 3H), 7.32 - 7.16 (m, 4H). ¹³C NMR (126 MHz, DMSO-*d₆*) δ 171.79, 161.51, 144.36, 136.52, 131.79, 130.58, 128.81, 128.36, 127.28, 126.96, 117.41, 114.59, 111.73. MS (ESI⁺): *m*/*z* 263.02 ([M+Na]⁺). HRMS (ESI⁻): *m*/*z* calcd for C₁₅H₁₂O₃-H⁺: 239.0714, found: 239.0714 ([M-H]⁻).

**2-Hydroxy-4-(phenylethynyl)benzoic acid (compound 26).** Preparation according to general procedure **(c)** using 4-bromo-2-hydroxybenzoic acid (compound 54, 0.10 g, 0.46 mmol) and phenylacetylene (compound 66, 62 µL, 0.92 mmol) for 1 h. The product was purified by auto RP-CC (H₂O + 0.1 % AcOH to MeCN + 0.1 % AcOH = 5 % → 95 %). Yield: 81 %. Pale brown solid. ¹H NMR (500 MHz, DMSO-*d₆*) δ 7.81 (d, *J* = 8.1 Hz, 1H), 7.60 - 7.55 (m, 2H), 7.47 - 7.41 (m, 3H), 7.11 (d, *J =* 1.4 Hz, 1H), 7.08 (dd, *J* = 8.1, 1.5 Hz, 1H). ¹³C NMR (126 MHz, DMSO-*d₆*) δ 171.45, 160.93, 131.77, 130.86, 129.52, 129.16, 129.00, 122.36, 121.79, 119.60, 113.51, 92.16, 88.51. MS (ESI⁻): *m*/*z* 237.21 ([M-H]⁻). HRMS (ESI⁻): *m*/*z* calcd for C₁₅H₁₀O₃-H⁺: 237.0557, found: 237.0557 ([M-H]⁻).

**2-Hydroxy-4-methyl-5-(phenylethynyl)benzoic acid (compound 27).** Preparation according to general procedure **(c)** using 5-bromo-2-hydroxy-4-methylbenzoic acid (compound 65, 309 mg, 1.30 mmol) and phenylacetylene (compound 66, 291 µL, 2.60 mmol) for 2 h. The product was purified by auto RP-CC (H₂O + 0.1 % AcOH to MeOH + 0.1 % AcOH = 30 % → 95 %). Yield: 32 %. Beige solid. ¹H NMR (500 MHz, Acetone-*d₆*) δ 8.00 (s, 1H), 7.57 (dd, *J* = 7.8, 1.7 Hz, 2H), 7.45 - 7.35 (m, 3H), 6.92 (s, 1H), 2.52 (s, 3H). ¹³C NMR (126 MHz, Acetone-*d₆*) δ 172.01, 162.89, 149.52, 134.66, 132.15, 129.44, 129.20, 124.25, 119.10, 115.20, 111.36, 92.74, 87.75, 21.28. MS (ESI⁻): *m*/*z* 251.03 ([M-H]⁻). HRMS (ESI⁻): *m*/*z* calcd for C₁₆H₁₂O₃-H⁺: 251.0714, found: 251.0713 ([M-H]⁻).

**2-Hydroxy-5-(4-methylphenylethynyl)benzoic acid (compound 28).** Preparation according to general procedure **(c)** using 5-bromo-2-hydroxybenzoic acid (compound 53, 306 mg, 1.38 mmol) and 1-ethynyl-4-methylbenzene (compound 67, 268 µL, 2.07 mmol) for 3 h. The product was purified by auto RP-CC using a gradient of H₂O and MeCN (H₂O + 0.1 % AcOH to MeCN + 0.1 % AcOH = 5 % → 95 %). Yield: 38 %. Beige solid. ¹H NMR (500 MHz, Acetone-*d₆*) δ 8.03 (d, *J* = 2.1 Hz, 1H), 7.66 (dd, *J* = 8.6, 2.1 Hz, 1H), 7.42 (d, *J* = 8.1 Hz, 2H), 7.21 (d, *J* = 8.0 Hz, 2H), 7.00 (d, *J* = 8.6 Hz, 1H), 2.35 (s, 3H). ¹³C NMR (126 MHz, Acetone-*d₆*) δ 172.05, 162.86, 139.35, 139.32, 134.33, 132.15, 130.08, 121.03, 118.76, 115.15, 113.59, 89.03, 88.28, 21.42. MS (ESI⁻): *m*/*z* 207.12 ([M-CO₂-H]⁻). HRMS (ESI⁻): *m*/*z* calcd for C₁₆H₁₂O₃-H⁺: 251.0714, found: 251.0711 ([M-H]⁻).

**5-[(4-Chlorophenyl)ethynyl]-2-hydroxybenzoic acid (compound 29).** Preparation according to general procedure **(c)** using 5-bromo-2-hydroxybenzoic acid (compound 53, 319 mg, 1.44 mmol) and 1-chloro-4-ethynylbenzene (compound 68, 296 mg, 2.16 mmol) for 3 h. The product was purified by auto RP-CC (H₂O + 0.1 % AcOH to MeCN + 0.1 % AcOH = 5 % → 95 %). Yield: 23 %. Pale yellow solid. ¹H NMR (500 MHz, Acetone-*d₆*) δ 8.05 (d, *J* = 2.2 Hz, 1H), 7.68 (dd, *J* = 8.6, 2.2 Hz, 1H), 7.59 - 7.52 (m, 2H), 7.47 - 7.41 (m, 2H), 7.01 (d, *J* = 8.6 Hz, 1H). ¹³C NMR (126 MHz, Acetone-*d₆*) δ 171.99, 163.15, 139.39, 134.68, 134.58, 133.78, 129.66, 122.87, 118.85, 114.55, 113.69, 90.07, 87.66. MS (ESI⁻): *m*/*z* 227.01 ([M-CO₂-H]⁻). HRMS (ESI⁻): *m*/*z* calcd for C₁₅H₁₀ClO₃-H⁺: 271.0167, found: 271.0162 ([M-H]⁻).

**2-Hydroxy-5-[(4-methoxyphenyl)ethynyl]benzoic acid (compound 30).** Preparation according to general procedure **(c)** using 5-bromo-2-hydroxybenzoic acid (compound 53, 312 mg, 1.41 mmol) and 1-ethynyl-4-methoxybenzene (compound 69, 366 µL, 2.82 mmol) for 2 h. The product was purified by auto RP-CC (H₂O + 0.1 % AcOH to MeCN + 0.1 % AcOH = 5 % → 95 %). Yield: 42 %. Pale brown solid. ¹H NMR (500 MHz, Acetone-*d₆*) δ 8.02 (d, *J* = 2.2 Hz, 1H), 7.65 (dd, *J* = 8.6, 2.2 Hz, 1H), 7.50 - 7.44 (m, 2H), 6.99 (d, *J* = 8.6 Hz, 1H), 6.98 - 6.93 (m, 2H), 3.82 (s, 3H). ¹³C NMR (126 MHz, Acetone-*d₆*) δ 172.03, 162.72, 160.80, 139.26, 134.18, 133.72, 118.73, 115.98, 115.39, 115.02, 113.50, 88.94, 87.49, 55.68. MS (ESI⁻): *m*/*z* 267.22 ([M-H]⁻). HRMS (ESI⁻): *m*/*z* calcd for C₁₆H₁₂O₄-H⁺: 267.0663, found: 267.0656 ([M-H]⁻).

**2-Hydroxy-5-[(4-hydroxyphenyl)ethynyl]benzoic acid (compound 31).** Preparation according to general procedure **(e)** using compound 30 (0.16 g, 0.57 mmol) for 1 h. The product was purified by auto RP-CC (H₂O + 0.1 % AcOH to MeOH + 0.1 % AcOH = 30 % → 95 %). Yield: 43 %. Pale yellow solid. ¹H NMR (500 MHz, Acetone-*d₆*) δ 8.01 (d, *J* = 2.2 Hz, 1H), 7.64 (dd, *J* = 8.6, 2.2 Hz, 1H), 7.42 - 7.38 (m, 2H), 6.99 (d, *J* = 8.6 Hz, 1H), 6.89 - 6.85 (m, 2H). ¹³C NMR (126 MHz, Acetone-*d₆*) δ 171.86, 162.43, 158.61, 139.26, 134.07, 133.86, 118.65, 116.39, 115.59, 114.82, 113.35, 89.27, 86.92. MS (ESI⁻): *m*/*z* 253.23 ([M-H]⁻). HRMS (ESI⁻): *m*/*z* calcd for C₁₅H₁₀O₄-H⁺: 253.0506, found: 253.0503 ([M-H]⁻).

**2-Hydroxy-5-(3-methylphenylethynyl)benzoic acid (compound 32).** Preparation according to general procedure (c) using 5-bromo-2-hydroxybenzoic acid (compound 53, 306 mg, 1.38 mmol) and 1-ethynyl-3-methylbenzene (compound 70, 363 µL, 2.76 mmol) for 3 h. The product was purified by auto RP-CC (H₂O + 0.1 % AcOH to MeCN + 0.1 % AcOH = 5 % → 95 %). Yield: 35 %. Ochre colored solid. ¹H NMR (500 MHz, Acetone-*d₆*) δ 8.05 (d, *J* = 2.0 Hz, 1H), 7.67 (dd, *J* = 8.6, 2.0 Hz, 1H), 7.36 (s, 1H), 7.33 (d, *J* = 7.7 Hz, 1H), 7.28 (t, *J* = 7.6 Hz, 1H), 7.20 (d, *J =* 7.6 Hz, 1H), 7.00 (d, *J* = 8.6 Hz, 1H), 2.34 (s, 3H). ¹³C NMR (126 MHz, Acetone-*d₆*) δ 172.04, 162.93, 139.34, 139.07, 134.42, 132.71, 130.04, 129.31, 123.90, 118.78, 115.00, 113.64, 89.05, 88.61, 21.17. MS (ESI⁻): *m*/*z* 207.10 ([M-CO₂-H]⁻). HRMS (ESI⁻): *m*/*z* calcd for C₁₆H₁₂O₃-H⁺: 251.0714, found: 251.0713 ([M-H]⁻).

**5-[(3-Chlorophenyl)ethynyl]-2-hydroxybenzoic acid (compound 33).** Preparation according to general procedure (c) using 5-bromo-2-hydroxybenzoic acid (compound 53, 306 mg, 1.38) and 1-chloro-3-ethynylbenzene (compound 71, 347 µL, 2.76 mmol) for 2 h. The product was purified by auto RP- CC (H₂O + 0.1 % AcOH to MeCN + 0.1 % AcOH = 5 % → 95 %). Yield: 22 %. Ochre colored solid. ¹H NMR (500 MHz, Acetone-*d₆*) δ 8.07 (d, *J* = 2.1 Hz, 1H), 7.69 (dd, *J* = 8.6, 2.1 Hz, 1H), 7.56 (s, 1H), 7.52 - 7.46 (m, 1H), 7.45 - 7.37 (m, 2H), 7.01 (d, *J* = 8.6 Hz, 1H). ¹³C NMR (126 MHz, Acetone-*d₆*) δ 171.95, 163.25, 139.47, 134.73, 131.75, 131.10, 130.66, 129.33, 126.01, 118.86, 114.34, 113.66, 90.35, 87.34. MS (ESI⁻): *m*/*z* 227.03 ([M-CO₂-H]⁻). HRMS (ESI⁻): *m*/*z* calcd for C₁₅H₉ClO₃-H⁺: 271.0167, found: 271.0167 ([M-H]⁻).

**2-Hydroxy-5-[(3-methoxyphenyl)ethynyl}benzoic acid (compound 34).** Preparation according to general procedure (c) using 5-bromo-2-hydroxybenzoic acid (compound 53, 316 mg, 1.43 mmol) and 1-ethynyl-3-methoxybenzene (compound 72, 363 µL, 2.86 mmol) for 2 h. The product was purified by auto RP- CC (H₂O + 0.1 % AcOH to MeCN + 0.1 % AcOH = 5 % → 95 %). Yield: 40 %. Pale brown solid. ¹H NMR (500 MHz, Acetone-*d₆*) δ 8.06 (d, *J* = 2.2 Hz, 1H), 7.68 (dd, *J* = 8.6, 2.2 Hz, 1H), 7.30 (t, *J* = 8.0 Hz, 1H), 7.14 - 7.07 (m, 2H), 7.01 (d, *J* = 8.6 Hz, 1H), 6.96 (dd, *J* = 8.3, 2.6 Hz, 1H), 3.83 (s, 3H). ¹³C NMR (126 MHz, Acetone-*d₆*) δ 171.96, 162.99, 160.60, 139.43, 134.51, 130.50, 125.08, 124.61, 118.82, 117.08, 115.68, 114.89, 113.57, 88.89, 88.70, 55.65. MS (ESI⁻): *m*/*z* 267.22 ([M-H]⁻). HRMS (ESI⁻): *m*/*z* calcd for C₁₆H₁₂O₄-H⁺: 267.0663, found: 267.0656 ([M-H]⁻).

**2-Hydroxy-5-[(3-hydroxyphenyl)ethynyl]benzoic acid (compound 35).** Preparation according to general procedure **(e)** using compound 34 (0.15 g, 0.57 mmol) for 1 h. The product was purified by auto RP-CC (H₂O + 0.1 % AcOH to MeOH + 0.1 % AcOH = 30 % → 95 %). Yield: 16 %. Brown solid. ¹H NMR (500 MHz, Acetone-*d₆*) δ 11.29 (s, 1H), 8.58 (s, 1H), 8.04 (d, *J* = 2.2 Hz, 1H), 7.68 (dd, *J* = 8.6, 2.2 Hz, 1H), 7.23 (t, *J =* 7.9 Hz, 1H), 7.06 - 6.98 (m, 3H), 6.88 (ddd, *J* = 8.3, 2.4, 0.8 Hz, 1H). ¹³C NMR (126 MHz, Acetone-*d₆*) δ 171.99, 162.98, 158.24, 139.45, 134.42, 130.57, 124.98, 123.65, 118.82, 118.73, 116.80, 114.97, 113.51, 88.95, 88.39. MS (ESI⁻): *m*/*z* 209.10 ([M-CO₂-H]⁻). HRMS (ESI⁻): *m*/*z* calcd for C₁₅H₁₀O₄-H⁺: 253.0506, found: 253.0504 ([M-H]⁻).

**2-Hydroxy-5-(2-methylphenylethynyl)benzoic acid (compound 36).** Preparation according to general procedure **(c)** using 5-bromo-2-hydroxybenzoic acid (compound 53, 314 mg, 1.42 mmol) and 1-ethynyl-2-methylbenzene (compound 73, 362 µL, 2.84 mmol) for 2 h. The product was purified by auto RP-CC (H₂O + 0.1 % AcOH to MeCN + 0.1 % AcOH = 5 % → 95 %). Yield: 57 %. Pale brown solid. ¹H NMR (500 MHz, Acetone-*d₆*) δ 11.10 (bs, 1H), 8.07 (d, *J = 2.1* Hz, 1H), 7.70 (dd, *J* = 8.6, 2.1 Hz, 1H), 7.49 (d, *J = 7.5* Hz, 1H), 7.31 - 7.23 (m, 2H), 7.23 - 7.17 (m, 1H), 7.01 (d, *J* = 8.6 Hz, 1H), 2.49 (s, 3H). ¹³C NMR (126 MHz, Acetone-*d₆*) δ 172.01, 162.97, 140.64, 139.38, 134.29, 132.50, 130.39, 129.28, 126.62, 123.75, 118.81, 115.17, 113.57, 93.04, 87.75, 20.79. MS (ESI⁻): *m*/*z* 251.27 ([M-H]⁻). HRMS (ESI⁻): *m*/*z* calcd for C₁₆H₁₂O₃-H⁺: 251.0714, found: 251.0713 ([M-H]⁻).

**5-[(2-Chlorophenyl)ethynyl]-2-hydroxybenzoic acid (compound 37).** Preparation according to general procedure **(c)** using 5-bromo-2-hydroxybenzoic acid (compound 53, 306 mg, 1.38 mmol) and 1-Chloro-2-ethynylbenzene (compound 74, 336 µL, 2.76 mmol) for 2 h. The product was purified by auto RP-CC (H₂O + 0.1 % AcOH to MeCN + 0.1 % AcOH = 5 % → 95 %). Yield: 27 %. Pale brown solid. ¹H NMR (500 MHz, Acetone-*d₆*) δ 11.16 (bs, 1H), 8.10 (d, *J = 2.1* Hz, 1H), 7.72 (dd, *J* = 8.6, 2.2 Hz, 1H), 7.64 (dd, *J* = 7.3, 2.0 Hz, 1H), 7.53 (dd, *J* = 7.8, 1.4 Hz, 1H), 7.43 - 7.34 (m, 2H), 7.04 (d, *J* = 8.6 Hz, 1H). ¹³C NMR (126 MHz, Acetone-*d₆*) δ 171.93, 163.34, 139.49, 136.01, 134.63, 134.14, 130.65, 130.21, 127.91, 123.79, 118.95, 114.46, 113.67, 94.25, 85.63. MS (ESI⁻): *m*/*z* 271.18 ([M-H]⁻). HRMS (ESI⁻): *m*/*z* calcd for C₁₅H₁₀ClO₃-H⁺: 271.0167, found: 271.0163 ([M-H]⁻).

**2-Hydroxy-5-[(2-methoxyphenyl)ethynyl]benzoic acid (compound 38).** Preparation according to general procedure **(c)** using 5-bromo-2-hydroxybenzoic acid (compound 53, 312 mg, 1.41 mmol) and 1-ethynyl-2-methoxybenzene (compound 75, 370 µL, 2.82 mmol) for 2 h. The product was purified by auto RP-CC (H₂O + 0.1 % AcOH to MeCN + 0.1 % AcOH = 5 % → 95 %). Yield: 49 %. Pale brown solid. ¹H NMR (500 MHz, Acetone-*d₆*) δ 8.06 (d, *J* = 2.2 Hz, 1H), 7.69 (dd, *J* = 8.6, 2.2 Hz, 1H), 7.49 (dd, *J* = 7.6, 1.7 Hz, 1H), 7.40 - 7.34 (m, 1H), 7.07 (d, *J =* 8.3 Hz, 1H), 7.03 (d, *J* = 8.6 Hz, 1H), 6.97 (td, *J* = 7.5, 0.8 Hz, 1H), 3.92 (s, 3H). ¹³C NMR (126 MHz, Acetone-*d₆*) δ 172.02, 162.81, 161.06, 139.35, 134.25, 134.00, 130.81, 121.24, 118.76, 115.53, 113.53, 113.18, 111.92, 92.62, 85.70, 56.10. MS (ESI⁻): m/z 267.22 ([M-H]⁻). HRMS (ESI⁻): *m*/*z* calcd for C₁₆H₁₂O₄-H⁺: 267.0663, found: 267.0657 ([M-H]⁻).

**2-Hydroxy-5-[(2-hydroxyphenyl)ethynyl]benzoic acid (compound 39).** Preparation according to general procedure **(e)** using compound 38 (87 mg, 0.32 mmol) for 1 h. The product was purified by automated reversed phase CC using a gradient of H₂O and MeOH containing additives of 0.1 % acetic acid. (MeOH 30 % to 95 %). Yield: 30 %. Pale yellow solid. ¹H NMR (500 MHz, Acetone-*d₆*) δ 11.28 (s, 1H), 8.51 (s, 1H), 8.09 (d, *J* = 2.2 Hz, 1H), 7.70 (dd, *J* = 8.6, 2.2 Hz, 1H), 7.43 (dd, *J* = 7.7, 1.6 Hz, 1H), 7.27 - 7.20 (m, 1H), 7.01 (d, *J* = 8.6 Hz, 1H), 6.95 (dd, *J* = 8.3, 0.6 Hz, 1H), 6.87 (td, *J* = 7.6, 0.9 Hz, 1H). ¹³C NMR (126 MHz, Acetone-*d₆*) δ 172.05, 162.87, 158.85, 139.43, 134.39, 133.57, 130.85, 120.54, 118.68, 116.51, 115.37, 113.45, 111.16, 93.38, 85.01. MS (ESI⁻): *m*/*z* 253.19 ([M-H]⁻). HRMS (ESI⁻): *m*/*z* calcd for C₁₅H₉O₄: 253.0506, found: 253.0505 ([M-H]⁻).

**2-Hydroxy-5-(4-methylphenethyl)benzoic acid (compound 40).** Preparation according to general procedure **(d)** using compound 28 (0.15 g, 0.61 mmol) for 2 h. Yield: 100 %. Beige solid. ¹H NMR (500 MHz, Acetone-*d₆*) δ 10.94 (s, 1H), 7.73 (d, *J* = 2.3 Hz, 1H), 7.37 (dd, *J* = 8.5, 2.3 Hz, 1H), 7.10 (d, *J =* 8.1 Hz, 2H), 7.06 (d, *J* = 8.0 Hz, 2H), 6.86 (d, *J* = 8.5 Hz, 1H), 2.92 - 2.80 (m, 4H), 2.27 (s, 3H). ¹³C NMR (126 MHz, Acetone-*d₆*) δ 172.72, 161.34, 139.35, 137.19, 135.88, 133.44, 130.61, 129.71, 129.24, 117.93, 112.72, 38.17, 37.57, 21.02. MS (ESI⁻): *m*/*z* 255.02 ([M-H]⁻). HRMS (ESI⁻): *m*/*z* calcd for C₁₆H₁₆O₃-H⁺: 255.1027, found: 255.1027 ([M-H]⁻).

**2-Hydroxy-5-(3-methoxyphenethyl)benzoic acid (compound 41).** Preparation according to general procedure **(d)** using compound 34 (0.13 g, 0.50 mmol) for 1 h. Yield: 100 %. Ochre colored solid. ¹H NMR (500 MHz, Acetone-*d₆*) δ 10.93 (s, 1H), 7.74 (d, *J* = 2.3 Hz, 1H), 7.39 (dd, *J* = 8.5, 2.3 Hz, 1H), 7.17 (t, *J* = 7.8 Hz, 1H), 6.87 (d, *J* = 8.5 Hz, 1H), 6.83 - 6.78 (m, 2H), 6.74 (dd, *J* = 8.5, 2.4 Hz, 1H), 3.75 (s, 3H), 2.93 - 2.83 (m, 4H). ¹³C NMR (126 MHz, Acetone-*d₆*) δ 172.71, 161.36, 160.74, 144.04, 137.21, 133.39, 130.64, 130.05, 121.60, 117.95, 115.01, 112.73, 112.19, 55.32, 38.63, 37.37. MS (ESI⁻): *m*/*z* 271.24 ([M-H]⁻). HRMS (ESI⁻): *m*/*z* calcd for C₁₆H₁₆O₄-H⁺: 271.0976, found: 271.0970 ([M-H]⁻).

**2-Hydroxy-5-(2-methylphenethyl)benzoic acid (compound 42).** Preparation according to general procedure **(d)** using compound 36 (0.24 g, 0.94 mmol) for 1 h. Yield: 95 %. Pale brown solid. ¹H NMR (500 MHz, Acetone-*d₆*) δ 10.95 (s, 1H), 7.74 (d, *J* = 2.3 Hz, 1H), 7.39 (dd, *J* = 8.5, 2.3 Hz, 1H), 7.18 - 7.05 (m, 4H), 6.88 (d, *J* = 8.5 Hz, 1H), 2.92 - 2.86 (m, 2H), 2.86 - 2.80 (m, 2H), 2.29 (s, 3H). ¹³C NMR (126 MHz, Acetone-*d₆*) δ 172.71, 161.40, 140.53, 137.19, 136.57, 133.52, 130.90, 130.58, 129.84, 126.88, 126.75, 117.98, 112.75, 36.33, 36.03, 19.33. MS (ESI⁻): *m*/*z* 255.27 ([M-H]⁻). HRMS (ESI⁻): *m*/*z* calcd for C₁₆H₁₆O₃-H⁺: 255.1027, found: 255.1028 ([M-H]⁻).

**5-[(3,4-Dichlorophenyl)ethynyl]-2-hydroxybenzoic acid (compound 43).** Preparation according to general procedure **(c)** using 5-bromo-2-hydroxybenzoic acid (compound 53, 330 mg, 1.47 mmol) and 1,2-dichloro-4-ethynylbenzene (compound 76, 520 mg, 2.95 mmol) for 2 h. The product was purified by auto RP-CC (H₂O + 0.1 % AcOH to MeOH + 0.1 % AcOH = 30 % → 95 %). Yield: 10 %. Beige solid. ¹H NMR (500 MHz, Acetone-*d₆*) δ 8.08 (d, *J* = 1.8 Hz, 1H), 7.75 (d, *J* = 1.9 Hz, 1H), 7.71 (dd, *J* = 8.6, 2.1 Hz, 1H), 7.61 (d, *J* = 8.3 Hz, 1H), 7.52 (dd, *J* = 8.3, 1.9 Hz, 1H), 7.03 (d, *J* = 8.6 Hz, 1H). ¹³C NMR (126 MHz, Acetone-*d₆*) δ 171.97, 163.43, 139.48, 134.82, 133.76, 132.95, 132.83, 132.01, 131.70, 124.57, 118.94, 114.08, 113.78, 91.24, 86.46. MS (ESI⁻): *m*/*z* 261.03 ([M-CO₂-H]⁻). HRMS (ESI⁻): *m*/*z* calcd for C₁₅H₈Cl₂O₃-H⁺: 304.9778, found: 304.9754 ([M-H]⁻).

**5-[(2,4-Dichlorophenyl)ethynyl]-2-hydroxybenzoic acid (compound 44).** Preparation according to general procedure **(c)** using 5-bromo-2-hydroxybenzoic acid (compound 53, 306 mg, 1.38 mmol) and 2,4-dichlorophenylacetylene (compound 77, 366 mg, 2.07 mmol) for 3 h. The product was purified by auto RP-CC (H₂O + 0.1 % AcOH to MeOH + 0.1 % AcOH = 30 % → 95 %). Yield: 14 %. Rust brown solid. ¹H NMR (500 MHz, Acetone-*d₆*) δ 8.08 (s, 1H), 7.71 (d, *J* = 8.2 Hz, 1H), 7.65 (d, *J* = 8.4 Hz, 1H), 7.60 (s, 1H), 7.41 (d, *J* = 8.3 Hz, 1H), 7.03 (d, *J* = 8.6 Hz, 1H). ¹³C NMR (126 MHz, Acetone-*d₆*) δ 171.91, 163.51, 139.49, 136.89, 135.12, 135.03, 134.73, 129.96, 128.32, 122.75, 118.98, 114.10, 113.72, 95.37, 84.67. MS (ESI⁻): *m*/*z* 305.14 ([M-H]⁻). HRMS (ESI⁻): *m*/*z* calcd for C₁₅H₈Cl₂O₃-H⁺: 304.9778, found: 304.9756 ([M-H]⁻).

**5-[(2,4-Dimethylphenyl)ethynyl]-2-hydroxybenzoic acid (compound 45).** Preparation according to general procedure **(c)** using 5-bromo-2-hydroxybenzoic acid (compound 53, 366 mg, 1.65 mmol) and compound 63 (323 mg, 2.48 mmol) for 3 h. The product was purified by auto RP-CC (H₂O + 0.1 % AcOH to MeOH + 0.1 % AcOH = 30 % → 95 %). Yield: 11 %. Beige solid. ¹H NMR (500 MHz, Acetone-*d₆*) δ 8.04 (d, *J* = 2.1 Hz, 1H), 7.68 (dd, *J* = 8.6, 2.2 Hz, 1H), 7.37 (d, *J* = 7.8 Hz, 1H), 7.10 (s, 1H), 7.05 - 6.98 (m, 2H), 2.45 (s, 3H), 2.31 (s, 3H). ¹³C NMR (126 MHz, Acetone-*d₆*) δ 172.07, 162.87, 140.50, 139.33, 134.16, 132.45, 131.17, 127.37, 120.77, 118.80, 115.41, 113.57, 92.30, 87.95, 21.37, 20.71. MS (ESI⁻): *m*/*z* 265.30 ([M-H]⁻). HRMS (ESI⁻): *m*/*z* calcd for C₁₇H₁₄O₃-H⁺ 265.0870, found: 265.0856 ([M-H]⁻).

### 5-[(2-Chloro-4-methylphenyl)ethynyl]-2-hydroxybenzoic acid (compound 46).

Preparation according to general procedure **(c)** using 5-bromo-2-hydroxybenzoic acid (compound 53, 504 mg, 2.28 mmol) and compound 64 (514 mg, 3.41 mmol) for 2 h. The product was purified by auto RP-CC (H₂O + 0.1 % AcOH to MeOH + 0.1 % AcOH = 30 % → 95 %). Yield: 17 %. Beige solid. ¹H NMR (500 MHz, Acetone-*d₆*) δ 8.07 (d, *J* = 1.8 Hz, 1H), 7.69 (dd, *J* = 8.5, 1.7 Hz, 1H), 7.50 (d, *J* = 7.9 Hz, 1H), 7.34 (s, 1H), 7.17 (d, *J* = 7.8 Hz, 1H), 7.02 (d, *J* = 8.6 Hz, 1H), 2.36 (s, 3H). ¹³C NMR (126 MHz, Acetone-*d₆*) δ 171.96, 163.20, 141.40, 139.39, 135.75, 134.50, 133.82, 130.61, 128.70, 120.76, 118.88, 114.67, 113.66, 93.44, 85.75, 21.12. MS (ESI⁻): *m*/*z* 285.21 ([M-H]⁻). HRMS (ESI⁻): *m*/*z* calcd for C₁₆H₁₁ClO₃-H⁺: 285.0324, found: 285.0324 ([M-H]⁻).

### Protein Preparation and Crystallography

***Preparation of recombinant HNF4α LBD.*** The gene encoding the HNF4α protein (UniProt accession number: P41235, amino acids 147-377) was cloned into the pMAL-c5e vector downstream of a maltose-binding protein (MBP), an His8 tag, and a TEV cleavage site. For protein expression, BL21 cells were transformed with the expression vector. Overnight cultures were inoculated into 2 L of fresh TB medium supplemented with 100 µg/mL ampicillin. Expression was induced at an OD600 of 1.4 by adding 0.5 mM IPTG, followed by incubation at 18 °C for 16 hours. After expression, cells were harvested by centrifugation at 8000 g, then resuspended in a buffer containing 50 mM Tris (pH 7.4), 500 mM NaCl, 30 mM imidazole, 1 mM TCEP, and 1× Pierce^{™} protease inhibitor cocktail. Cells were lysed via sonication, and cellular debris was removed by centrifugation. The protein was purified using an IMAC column. Delipidation (optional) was performed using Phenyl Sepharose 6 Fast Flow resin as previously described³⁰. The HNF4α protein was cleaved from the MBP-His8-HNF4α fusion by overnight digestion with TEV protease at 4 °C. The cleaved MBP-His tag was removed using a reverse IMAC column. Finally, the target protein was purified by size exclusion chromatography (SEC) on a HiLoad 16/600 Superdex 200 column.

***Crystallography.*** The peak protein fractions from SEC were combined and concentrated to 15 mg/mL. A 5-fold molar excess of compound 23 was added to the protein, and the complex was incubated at 4 °C for 16 hours, followed by the addition of a 3-fold molar excess of the GRIP-1 cofactor peptide (KHKILHRLLQD). The complex was crystallized by hanging drop vapor diffusion at 16 °C in 16% PEG 3350, 8% ethylene glycol, and 0.1 M Bis-Tris propane (pH 8.5). The crystals grew over 2-3 weeks. They were cryoprotected with an additional 25% ethylene glycol and flash-frozen in liquid nitrogen. X-ray diffraction data were collected on beamline ID23-2 at the European Synchrotron Radiation Facility (ESRF)³¹. The data showed moderate anisotropic behavior and were processed by autoPROC³² using the STARANISO³³ server. The structure was solved by molecular replacement using 3fs1 as the search model. Iterative cycles of refinement and manual model building were performed using PHENIX³⁴ and COOT³⁵, respectively. X-Ray Diffraction Data and Refinement Statistics are presented in Table S1. LC-MS/MS analysis confirmed presence of the ligand of compound 23 in the crystal (Figure S1).

***Determination of compound 23 from HNF4α LBD co-crystal.*** To confirm that the observed positive electron density belonged to compound 23, the crystal was picked from the drop, dipped in crystallization buffer, and then dissolved in 300 µL of 0.1% formic acid and acetonitrile (50/50, v/v). A 100 µL aliquot of this final sample was then injected into the LC-MS. LC-ESI-MS/MS was performed using an API 5500 QTRAP triple quadrupole mass spectrometer with a TurboV-ion source (Sciex, Darmstadt, Germany) coupled to an Agilent 1260 HPLC system (Agilent, Waldbronn, Germany) and a SIL-20A/HT autosampler (Shimadzu, Duisburg, Germany) under control of Analyst 1.6 (Sciex). As stationary phase a TriArt C18 (3 µm, 50 mm x 2 mm, YMC, protected with a 0.5 µm and a 0.2 µm frit) in combination with 0.1 % formic acid in water (A) and 0.1 % formic acid in acetonitrile (B) as mobile phase (A/B, 45/55, v/v) at a flow rate of 400 µL/min was used. After washing, the crystal was dissolved in 300 µL of 0.1 % formic acid and acetonitrile (50/50, v/v) and 100 µL of this final sample injected into the LC-MS.

### In vitro Characterization

***Hybrid reporter gene assays.*** HEK293T cells (American Type Culture Collection (ATCC), Manassas, Virginia, USA, #CRL-3216) were cultured to a max. confluence of 70-80 % at 37 °C and 5 % CO₂ in DMEM, high glucose (Thermo Fisher Scientific, Waltham, Massachusetts, USA, #41965039) supplemented with 10 % fetal bovine serum (Capricorn Scientific GmbH, Ebsdorfergrund, Germany, #FBS-11A), 1 mM sodium pyruvate (Thermo Fisher Scientific, #11360039), 100 U/mL penicillin, and 100 µg/mL streptomycin (Thermo Fisher Scientific, #15140122). The cells were seeded into 96-well plates (40,000 cell/well). After 20-24 h at 37 °C and 5 % CO₂, the medium was changed to Opti-MEM (Thermo Fisher Scientific #51985026) without supplements, and the cells were transiently transfected with pFR-Luc (Agilent Technologies, Santa Clara, California, USA, #219050) as reporter gene, pRL-SV40 (Promega, Fitchburg, Wisconsin, USA, #E2231) as internal control for normalization of transfection efficiency and cell growth and one pFA-CMV-hNR-LBD coding for the hinge region and ligand binding domain (LBD) of a human nuclear receptor (NR) of interest, or pECE-SV40-Gal4-VP16¹⁹ (gift from Lea Sistonen; Addgene plasmid #71728) as ligand-independent transcriptional inducer for control experiments using Lipofectamine LTX (Thermo Fisher Scientific, #15338100) according to manufacturer's protocol. Five hours after transfection, the cells were incubated with test compounds by changing the medium to Opti-MEM supplemented with penicillin (100 U/mL) and streptomycin (100 µg/mL) additionally containing 0.1 % DMSO and the respective test compound or 0.1 % DMSO alone as untreated control. Firefly (reporter) and renilla (control) luminescence were measured after 14-16 hours incubation using the Dual-Glo^{®} Luciferase Assay System (Promega, Fitchburg, Wisconsin, USA; #E2980) according to manufacturer's protocol on a Tecan Spark^{®} 10 M multimode microplate reader (Tecan Group AG, Männedorf, Switzerland). Relative light units (RLU) were calculated by dividing the firefly luminescence with renilla luminescence and multiplication by 1,000. By dividing the mean RLU of the test compound at a respective concentration with the mean RLU of the DMSO (0.1%) control, the fold activation was obtained. Experiments were performed in duplicates in at least three biologically independent repeats. For dose response curve fitting and calculation of EC₅₀/IC₅₀ values, the equation "[Agonist]/[Inhibitor] vs. response - variable slope (four parameters)" was used in GraphPad Prism 9 (version 9.5.1, GraphPad software, La Jolla, CA, USA). The following pFA-CMV-hNR-LBD plasmids^{28,29} and reference ligands were used: THRα (pFA-CMV-hTHRα-LBD, 1 µM T3), RARα (pFA-CMV-RARα-LBD, 1 µM all*-trans* retinoic acid (ATRA)), PPARα (pFA-CMV-hPPARα-LBD, 1 µM GW7647), PPARδ (pFA-CMV-hPPARβ-LBD, 1 µM L-165,041), PPARγ (pFA-CMV-hPPARγ-LBD, 1 µM rosiglitazone), LXRα (pFA-CMV-hLXRα-LBD, 1 µM T0901317), FXR (pFA-CMV-hFXR-LBD, 1 µM GW4064), VDR (pFA-CMV-hVDR-LBD, 1 µM calcitriol), PXR (pFA-CMV-hPXR-LBD, 10 µM CITCO), CAR (pFA-CMV-hCAR-LBD, 1 µM CITCO), HNF4α/γ (pFA-CMVhHNF4α/γ-LBD, 10 µM compound 4¹⁷), RXRα (pFA-CMV-hRXRα-LBD, 1 µM bexarotene).

***Isothermal Titration Calorimetry (ITC).*** ITC experiments were conducted on an Affinity ITC instrument (TA Instruments, New Castle, DE) at 25 °C with a stirring rate of 75 rpm. HNF4α LBD protein (with bound lipid (20 µM) or lipid-free (5 µM)) in buffer (20 mM Tris pH 7.5, 150 mM NaCl, 0.2 mM TCEP, 5% glycerol) containing 3% DMSO was titrated with compound 46 (100 µM or 50 µM in the same buffer containing 3% DMSO) in 26 injections (1x 1 µL, 25x 4 µL) with an injection interval of 120 s. As control experiments, the test compound was titrated to the buffer, and the buffer was titrated to the HNF4α LBD protein under otherwise identical conditions. Results were analyzed using NanoAnalyze software (version 3.11.0, TA Instruments, New Castle, DE) with independent binding models.

***Cytotoxicity assay.*** HepG2 cells were cultured at 37 °C and 5% CO₂ in DMEM high-glucose supplemented with sodium pyruvate (1 mM), penicillin (100 U/mL), streptomycin (100 µg/mL), and 10% fetal calf serum (FCS) and seeded at a density of 30,000 cells/well. After 24 h, the cells were treated with the solubilized (0.1% DMSO) test compounds in DMEM supplemented with FCS (0.2%) penicillin (100 U/mL) and streptomycin (100 µg/mL). Each sample was prepared in four repeats. Bexarotene (100µM) served as positive control. After incubation for 24 h, the medium was changed to DMEM without phenol red supplemented as described above and 10% water-soluble tetrazolium salt (WST-8, Cell Counting Kit-8, MedChemExpress) was added to assess metabolic activity. After 2 h, absorbance was measured at 450 nm using a Tecan Spark Cyto instrument (Tecan, Männedorf, Switzerland). Thereafter Live-or-Dye Nuc-Fix Red (Biotium, Inc., Fremont, CA, 1691 USA) dye was added to a final concentration of 0.05x. After incubation for 30 min, a total of 3 fluorescence images per well at 10X magnification were taken to detect Live-or-Dye-positive cells (Ex: 543-566 nm, Em: 580-611 nm) using the Tecan Spark Cyto. Reference readings for background correction and detection of auto-fluorescence at the same wavelengths were taken prior to staining. Before test compound administration, after the first medium exchange, 24 h after test compound administration, and after fluorescence imaging, cell confluence was assessed using the Tecan Spark Cyto to account for changes in cell confluence due to test compound administration and cell handling. Cell confluence, metabolic activity, and necrotic cell counts were normalized to the DMSO control.

## Claims

1. A modulator of hepatocyte nuclear factor 4 (HNF4), comprising one of the following generic chemical structures 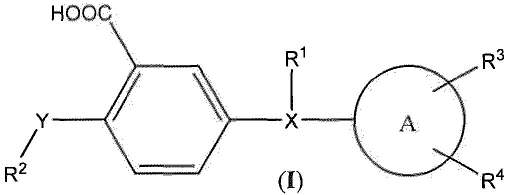 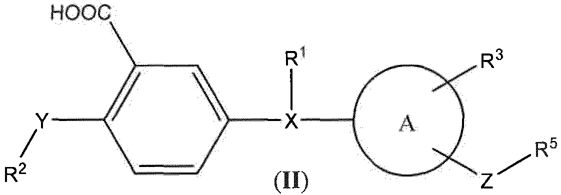 wherein
A is benzene or a heteroaromatic system,
X is C1-C5 alkyl, C3-C9 (bi)cycloalkyl, C2-C5 alkenyl, or C2-C5 alkynyl,
Y is -C(=O)-, -C(=O)NH-, -NHC(=O)-, -C-, -O-, -NH-, -N(alkyl)-, or -S-
Z is C1-C4 alkyl, -C(=O)-, -C(=O)NH-, -NHC(=O)-, -O-, -NH-, -N(alkyl)-, or -S-
R1 is -H, C1-C4 alkyl, or C1-C4 acyl;
R2 is -H, -OH, -NH(alkyl), C1-C4 alkyl, or C1-C4 acyl;
R3, R4, R5 is independently selected from -H, C1-C6 alkyl, C3-C6 cycloalkyl, C1-C6 haloalkyl, C1-C6 acyl, halo, phenyl optionally substituted, or heteroaryl optionally substituted
R3 and R4 or R3 and R5 optionally form a cyclic system.

2. The modulator of HNF4 according to claim 1, wherein
the heteroaromatic system is selected from the group consisting of pyridine, pyrimidine, pyridazine, pyrazine, furan, thiophene, pyrrole, oxazole, isoxazole, imidazole, pyrazole, triazole, thiazole, isothiazole;
X is C1-C5 alkyl, C3-C6 cycloalkyl, C4-C9 bicycloalkyl, C1-C5 alkenyl, C1-C5 alkynyl, each optionally containing -O-, -C(=O)-, -N(R1)-, or -C(=O)N(R1)-;
R3 and R4 or R3 and R5 optionally form a cyclic system.

3. The modulator of HNF4 according to claim 1 or claim 2, wherein the modulator is selected from the group consisting of:

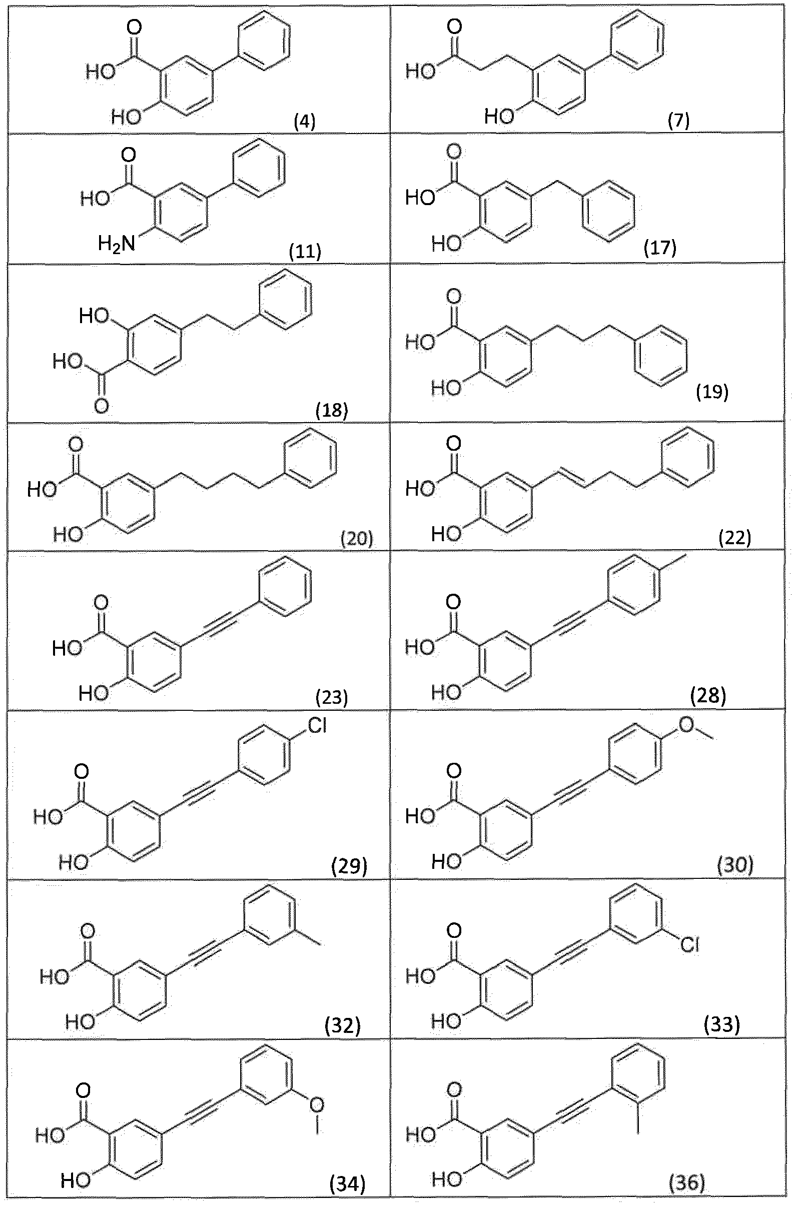

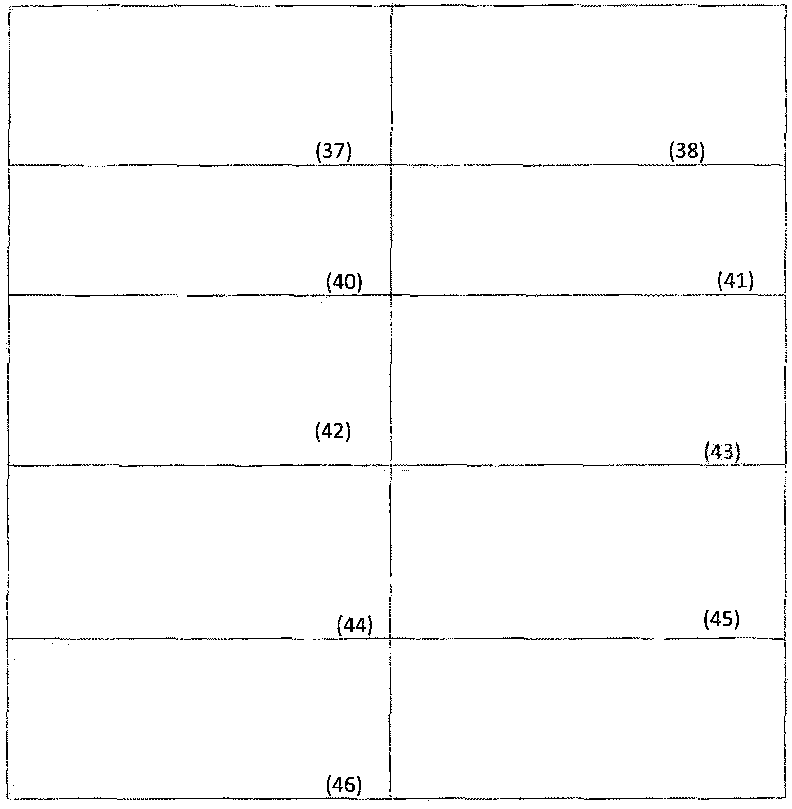

4. The modulator of HNF4 according to claim 1, wherein the HNF4 modulator is a compound comprising the formula 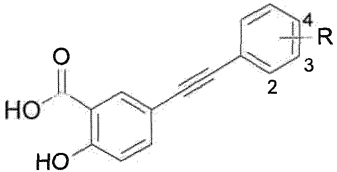 wherein
R2 is -H, -CH₃, -Cl, -OCH₃;
R3 is -H, -CH₃, -Cl, -OCH₃;
R4 is -H -CH₃, -Cl, -CH₃.

5. The modulator of HNF4 according to claim 4, wherein R3 is Cl and R4 is CI, or
R2 is Cl and R4 is Cl, or
R2 is CH₃ and R4 is CH₃, or
R2 is Cl and R4 is CH₃.

6. The modulator of HNF4 according to claim 4, wherein the compound is 5-[(2-Chloro-4-methylphenyl)ethynyl]-2-hydroxybenzoic acid having the structure

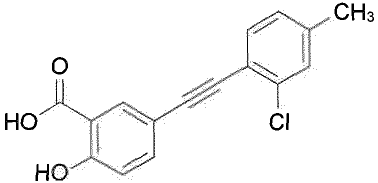

7. The modulator of HNF4 according to claim 1, wherein the modulator is an HNF4α. or HNF4γ agonist, or an HNF4α. or HNF4γ antagonist.

8. A modulator of hepatocyte nuclear factor 4 (HNF4) according to any one of claims 1 to 7 for use in the treatment or prevention of a disease that is associated with HNF4 malfunction or genetic mutations of the HNF4 gene.

9. The modulator of HNF4 for the use according to claim 8, wherein the disease is any one of such type 2 diabetes, maturity-onset diabetes of the young type 1 (MODY1), maturity-onset diabetes of the young type 3 (MODY3), neonatal hyperinsulinemic hypoglycemia, gastrointestinal disease, liver disease, pancreatic disease, gastrointestinal cancer, inflammatory bowel disease (IBD), atherosclerosis, metabolic dysfunction, metabolic syndrome, insulin resistance or lipid disorders.

10. A pharmaceutical composition, comprising a modulator of hepatocyte nuclear factor 4 (HNF4) according to any one of claims 1 to 7.

11. A pharmaceutical composition, comprising a modulator of hepatocyte nuclear factor 4 (HNF4) according to any one of claims 1 to 7 for the use in the treatment or prevention of a disease that is associated with HNF4 malfunction or genetic mutations of the HNF4 gene.

12. The pharmaceutical composition for the use according to claim 11, wherein the disease is associated with HNF4α malfunction or genetic mutations of the HNF4α gene.

13. The pharmaceutical composition for the use according to claim 11, wherein the disease is any one of type 2 diabetes, maturity-onset diabetes of the young type 1 (MODY1), maturity-onset diabetes of the young type 3 (MODY3), neonatal hyperinsulinemic hypoglycemia, gastrointestinal disease, liver disease, pancreatic disease, gastrointestinal cancer, inflammatory bowel disease (IBD), atherosclerosis or lipid disorders.

14. The pharmaceutical composition for the use according to claim 11 or claim 12, wherein the disease is type 2 diabetes or maturity-onset diabetes of the young type 1 (MODY1).

15. An in vitro method of analyzing the effects of hepatocyte nuclear factor 4 (HNF4) activity in a biological system, comprising the modulation of HNF4 activity by inserting a modulator as defined in any one of claims 1 to 7 into a cell.
